# FASCICULE DE BREVET EUROPEEN

(11) **EP 1 641 920 B1**
(45) Date de publication et mention de la délivrance du brevet: **17.03.2010**
(21) Numéro de dépôt: 04767632.5
(22) Date de dépôt: 08.07.2004
(51) Int. Cl.: C12N 9/10, C12N 9/90, C12N 15/54, C07K 1/14, C12Q 1/48

(54) **GLUTAMINE:FRUCTOSE-6-PHOSPHATE AMIDOTRANSFERASE (GFAT) COMPRENANT UNE ETIQUETTE DE PURIFICATION INTERNE, ET SON UTILISATION POUR LE CRIBLAGE DE COMPOSES**
EINEN INTERNEN REINIGUNGSMARKER ENTHALTENDE GLUTAMIN:FRUCTOSE-6-PHOSPHAT- AMIDOTRANSFERASE (GFAT) UND VERWENDUNG DAVON ZUM SCREENING VON VERBINDUNGEN
GLUTAMINE:FRUCTOSE-6-PHOSPHATE AMIDOTRANSFERASE (GFAT) COMPRISING AN INTERNAL PURIFICATION MARKER AND USE THEREOF FOR THE SCREENING OF COMPOUNDS

(30) Priorité: 08.07.2003 FR 0308350
(43) Date de publication de la demande: 05.04.2006
(73) Titulaire: CENTRE NATIONAL DE LA RECHERCHE SCIENTIFIQUE (CNRS), 75794 Paris Cedex 16 (FR)
(72) Inventeur: BADET-DENISOT, Marie-Ange, Juliette, Etiennette, F-91470 Forges-Les-Bains (FR); BADET, Bernard, François, F-91470 Forges-Les-Bains (FR)
(74) Mandataire: Grosset-Fournier, Chantal Catherine
(86) Numéro de dépôt international: PCT/FR2004/001800
(87) Numéro de publication internationale: WO 2005/005628

(56) Documents cités:
- WO-A-93/21330
- WO-A-97/07132
- WO-A-02/090535
- US-A- 5 710 248

## Description

La présente invention concerne une glutamine:fructose-6-phosphate amidotransférase modifié, purifiable rapidement et en quantités suffisantes pour le criblage de composés modifiant son activité.

Les glutamine:fructose-6-phosphate amidotransférases (GFAT), EC 2.6.1.16, également appelées glucosamine-6-phosphate synthases ou 2-déoxy-glucose-6-phosphate cétol isomérases, sont impliquées dans la voie de biosynthèse des hexosamines. La GFAT catalyse la première étape, limitante, de cette voie de biosynthèse selon la réaction :
L-Glutamine + fructose-6-phosphate → L-Glutamate + glucosamine-6-phosphate par transfert de l'azote amidique de la L-Glutamine sur la fonction cétone du fructose-6-phosphate. Les GFAT contrôlent donc le flux de glucose dans la voie des hexosamines, via le fructose-6-phosphate, et par conséquent la formation des hexosamines produites.

Une forme bactérienne recombinante de la GFAT, la glucosamine-6-phosphate synthase *d'Escherichia coli,* a été purifiée à homogénéité et étudiée de façon exhaustive. Les propriétés et le mécanisme enzymatique du transfert de l'amide ont notamment été largement décrits (pour revue Teplyakov et al., Nat. Prod. Rep. (2002) 19:60). En particulier, cette enzyme, dont la structure cristalline a été résolue (Teplyakov et al., J. Mol. Biol. (2001) 313:1093), est formée de deux domaines, l'un ayant une activité hydrolase (glutaminase) et l'autre une activité isomérase.

Par ailleurs, des GFAT d'eucaryotes ont été caractérisées, dont notamment celle de foie de rat (Huynh et al., Arch. Biochem. Biophys. (2000) 379:307) et celle de la levure *Candida albicans* (Milewsky et al., J. Biol. Chem. (1999) 274:4000).

Chez l'homme, des études préliminaires ont montré la présence d'une activité GFAT dans le foie (Ghosh et al., J. Biol. Chem. (1960) 235:1265). Plusieurs GFAT sont désormais connues. GFAT1, la forme principale, GFAT2, qui est exprimée préférentiellement dans le système nerveux central, et GFAT1Alt, une isoforme de GFAT1, exprimée essentiellement dans les muscles striés. Les séquences peptidiques de GFAT1 et GFAT2 possèdent 75% d'identités de séquences entre elles, et celles de GFAT1 et GFAT1Alt sont identiques excepté pour une insertion de 18 acides aminés dans la séquence de GFAT1Alt. Les séquences de GFAT sont donc très conservées chez l'homme, mais également entre les espèces, puisque les séquences peptidiques de la GFAT1 humaine et de la GFAT d'E. *coli* ou de la GFAT1 de souris présentent respectivement 35% et 99% d'identités.

Le gène de la GFAT1 humaine a été cloné en 1992 (McKnight et al., J. Biol. Chem. (1992) 267:25208). Il code une protéine de 77 kDa formée de deux domaines distincts (Teplyakov et al., Nat. Prod. Rep. (2002) 19:60).

L'augmentation de la production de l'UDP-NAc-GlcNH₂, le produit final de la voie de biosynthèse des hexosamines, et son accumulation dans les tissus ont récemment été impliquées dans le développement de la résistance à l'insuline (Marshall et al., FASEB J. (1991) 5:3031, Yki-Jarvinen et al., Diabetes (1996) 45:302, Thompson et al., J. Biol. Chem. (1997) 272: 7759, Hawkins et al., J. Clin. Invest. (1997) 99:2173, Robinson et al., Diabetes (1993) 42:1333, Daniels et al., J. Clin. Invest. (1995) 96:1235, Baron et al., J. Clin. Invest. (1995) 96:2792).

Ainsi, il a été montré qu'une augmentation du niveau cellulaire d'UDP-NAc-GlcNH₂ par une modeste surexpression de GFAT1, ou un apport de glucosamine exogène, peut induire une résistance à l'insuline à la fois in vivo et dans des adipocytes en culture (Robinson et al., Diabetes (1993) 42:1333, Daniels et al., J. Clin. Invest. (1995) 96:1235, Baron et al., J. Clin. Invest. (1995) 96:2792, Hebert et al., J. Clin. Invest. (1996) 98:930).

En effet, l'insuline active sa voie de transduction en se fixant à son recepteur, ce qui induit la translocation des transporteurs de glucose, tels que le récepteur GLUT4, stockés dans la cellule, vers la membrane, et augmente l'afflux de glucose. Le glucose entre ainsi dans la voie de la glycolyse et est converti en glucose-6-phosphate puis en fructose-6-phosphate. Lorsque l'afflux de glucose est excessif, le fructose-6-phosphate entre dans la voie de biosynthèse des hexosamines et est converti en glucosamine-6-phosphate par la GFAT. Plusieurs observations indiquent que les métabolites de la glucosamine-6-phosphate empêchent la translocation des recepteurs au glucose vers la membrane cellulaire, diminuant ainsi l'afflux du glucose cellulaire (Marshall et al., FASEB J. (1991) 5:3031, Giacarri et al., Diabetologia (1995) 38:518, Marshall et al., J. Biol. Chem. (1991) 266:4706, Paterson et al., Endocrinology (1995) 136:2809).

Le mécanisme par lequel les métabolites de la glucosamine-6-phosphate exercent leurs effets physiologiques n'est pas clair. Une hypothèse a cependant été proposée : une concentration cytosolique élevée d'UDP-NAc-GlcNH₂ entraînerait l'hyperglycosylation des sites de phosphorylation Ser ou Thr, conduisant de la sorte à l'arrêt de la voie de signalisation de l'insuline (Comer et al., J Biol. Chem (2000) 275:29179).

L'activité GFAT est donc considérée comme étant l"une des causes des hauts niveaux de glucose sanguin ; par ailleurs elle est connue pour être élevée chez les patients atteints de diabète sucré non-insulino dépendant ou diabète de type-II (Yki-Jarvinen et al., Diabetes (1996) 45:302).

L'obtention d'inhibiteurs de la GFAT permettrait d'abaisser la glycémie en particulier chez les individus atteints de pathologies liées à une hyperglycémie, telles que le diabète de type II, l'acidose et/ou la cétose diabétique, par exemple.

Des inhibiteurs de la GFAT de plante ou de champignon pourraient également permettre d'obtenir respectivement des fongicides et des herbicides.

Toutefois, malgré l'obtention de formes recombinantes de GFAT, l'instabilité des préparations enzymatiques obtenues, leur faible quantité, et leur niveau de purification insuffisant, n'ont pas permis d'obtenir des inhibiteurs efficaces de GFAT.

Un objet de l'invention est donc de fournir une GFAT modifiée dont l'activité est stable et pouvant être obtenue en grande quantité, avec un haut niveau de pureté et d'activité.

La présente invention concerne une protéine enzymatiquement active comprenant :
- une séquence de GFAT et au moins une séquence d'une étiquette de purification, la séquence de l'étiquette de purification étant insérée entre deux acide aminés consécutifs de la séquence de GFAT,
   lesdits acides aminés étant compris dans :
   - une séquence de GFAT correspondant à la séquence s'étendant entre les acides • aminés 30 à 80 de la GFAT d'Escherichia coli, ou
   - une séquence de GFAT correspondant à la séquence s'étendant entre les acides aminés 220 à 230 de la GFAT d'Escherichia coli.
      ou
- une séquence dérivant de la séquence précédente par suppression, insertion ou mutation d'au moins un acide aminé, sous réserve que ladite protéine présente une activité enzymatique, ou
- une séquence présentant au moins 35 %, notamment au moins 90 %, d'identité de séquence et/ou au moins 44 %, notamment au moins 95 %, de similarité des séquence avec l'une des séquences précédentes, sous réserve que ladite protéine présente une activité enzymatique.

On désigne par GFAT une enzyme de la classe E.C. 2.6.1.16 catalysant la réaction suivante :
L-Glutamine + fructose-6-phosphate → L-Glutamate + glucosamine-6-phosphate notamment dans les conditions expérimentales telles qu'elles sont décrites dans l'exemple qui suit ou dans Broschat et al., J. Biol. Chem. (2002) 277:14764.

GFAT est désignée sous le nom de glutamine:fructose-6-phosphate amidotransférase, ou également glucosamine-6-phosphate synthase ou 2-déoxy-glucose-6-phosphate cétol isomérase.

On désigne par « protéine enzymatiquement active » une protéine ayant une action catalytique.

Avantageusement, la protéine enzymatiquement active possède une activité GFAT.

On désigne par « étiquette de purification » une séquence peptidique susceptible de se lier spécifiquement à un ligand donné. Avantageusement, la liaison dudit ligand à l'étiquette de purification permet de former un complexe entre la protéine portant l'étiquette de purification et ledit ligand, ledit complexe pouvant être spécifiquement isolé.

Avantageusement, les étiquettes de purification selon l'invention ne sont pas placées en bout de chaîne peptidique, à l'extrémité N-terminale ou C-terminale, mais à l'intérieur de la chaîne peptidique.

On désigne par « identité de séquence » le pourcentage d'acides aminés identiques entre deux séquences alignées, notamment à l'aide d'algorithmes tels que celui défini par Altschul et al., Nucleic Acids Res. (1997) 25:3389, par exemple.

On désigne par « similarité de séquence » le pourcentage d'acides aminés similaires, c'est-à-dire d'acides aminés dont les chaînes latérales possèdent des propriétés physico-chimiques proches, entre deux séquence alignées, notamment à l'aide d'algorithmes tels que celui défini par Altschul et al., Nucleic Acids Res. (1997) 25:3389, par exemple.

La présente invention concerne en particulier une protéine telle que définie ci-dessus, dans laquelle la séquence de GFAT correspond à une séquence de GFAT de bactérie ou d'eucaryote, notamment de plante, de champignon ou d'animal, en particulier d'insecte ou de mammifère, plus particulièrement d'homme.

L'invention concerne plus particulièrement une protéine telle que définie ci-dessus, dans laquelle la séquence de l'étiquette de purification est insérée entre deux acides aminés consécutifs de la séquence de GFAT, lesdits acides aminés étant compris dans :
- une partie de la séquence de la GFAT correspondant et/ou étant homologue à la séquence s'étendant entre le feuillet β2 et le feuillet β3 de la GFAT d'*Escherichia coli,* ou
- une partie de la séquence de la GFAT correspondant et/ou étant homologue à la séquence s'étendant entre le feuillet β13 et le feuillet β14 de la GFAT *d'Escherichia coli.*

La structure de la GFAT *d'Escherichia coli* est décrite en particulier par Teplyakov et al., J. Mol. Biol. (2001) 313:1093 (protéine entière), par Isupov et al., Structure (1996) 4:801 (domaine glutaminase) et par Teplyakov et al., Structure (1998) 6:1047 (domaine isomérase). La structure de la protéine complète est notamment consultable à l'aide du fichier de coordonnées atomiques 1JXA déposé auprès de la *Protein Data Bank* (http://www.pdb.org).

La séquence peptidique de la GFAT d'*E. coli* est définie par SEQ ID NO : 13.

La séquence s'étendant entre le feuillet β2 et le feuillet β3 correspond à la séquence s'étendant approximativement entre les acides aminés 30 à 80 de la GFAT d'*E. coli,* situés dans le domaine glutaminase.

La séquence s'étendant entre le feuillet β13 et le feuillet β14 correspond à la séquence s'étendant approximativement entre les acides aminés 220 à 230 de la GFAT d'*E. coli*, situés dans le domaine glutaminase.

Selon un mode de réalisation particulier, l'invention concerne donc une protéine telle que définie ci-dessus, dans laquelle la séquence de l'étiquette de purification est insérée entre deux acides aminés consécutifs de la séquence de GFAT, lesdits acides aminés étant compris dans :
- une partie de la séquence de la GFAT correspondant et/ou étant homologue à la séquence s'étendant approximativement entre les acides aminés 30 à 80 de la GFAT d'*Escherichia coli,* ou
- une partie de la séquence de la GFAT correspondant et/ou étant homologue à la séquence s'étendant approximativement entre les acides aminés 220 à 230 de la GFAT *d'Escherichia coli.*

L'identification des parties de séquences d'une GFAT correspondant et/ou étant homologue à des structures secondaires de la GFAT d'E. *coli* peut être obtenue en alignant la séquence de ladite GFAT avec celle de la GFAT d'E. *coli,* notamment à l'aide d'un algorithme tel que celui défini par Altschul et al., Nucleic Acids Res. (1997) 25:3389 ou à l'aide du logiciel Clustal W, bien connu de l'homme de l'art et décrit par Thompson et al., Nucleic Acids Res. (1994) 22: 4673-4680, par exemple.

En particulier, deux séquences ou parties de séquences sont dites homologues si le pourcentage d'identité entre ces deux séquences ou parties de séquences est supérieur à environ 35% et/ou si le pourcentage de similarité entre ces deux séquences ou parties de séquences est supérieur à environ 44%.

Plus particulièrement, deux séquences ou parties de séquences sont dites homologues si elles sont susceptibles de s'hybrider dans des conditions stringentes, telles que les conditions suivantes : formamide 50%, NaCl 0,75 mol/1, citrate de sodium 0,75 mmol/1, sodium dodécyl sulfate 1%, pH 7, 42°C.

Selon un autre mode de réalisation préféré, l'invention concerne une protéine telle que définie ci-dessus, dans laquelle la séquence de l'étiquette de purification est insérée entre deux acides aminés consécutifs de la séquence d'une GFAT humaine, lesdits acides aminés étant compris entre les acides aminés 40 à 50 et/ou 290 à 330, de ladite séquence de GFAT humaine.

Les acides aminés 40 à 50 de ladite séquence de GFAT humaine correspondent et/ou sont homologues à la partie de la séquence de la GFAT d'*E. coli* s'étendant entre le feuillet β2 et le feuillet β3, c'est-à-dire à la séquence s'étendant approximativement entre les acides aminés 30 à 80 de la GFAT d'*E*. *coli.*

Les acides aminés 290 à 330 de ladite séquence de GFAT humaine correspondent et/ou sont homologues à la partie de la séquence de la GFAT d'E. *coli* s'étendant entre le feuillet β13 et le feuillet β14, c'est-à-dire à la séquence s'étendant approximativement entre les acides aminés 220 à 230 de la GFAT d'*E*. *coli.*

L'invention concerne notamment une protéine ci-dessus, dans laquelle la séquence de GFAT correspond à :
- SEQ ID NO : 2, correspondant à la séquence de la GFAT1 humaine,
- SEQ ID NO : 4, correspondant à la séquence de la GFAT2 humaine,
- SEQ ID NO : 6, correspondant à la séquence de la GFAT1Alt humaine.

La séquence de la GFAT1 humaine est notamment décrite dans McKnight et al., J. Biol. Chem. (1992) 267:25208, et correspond à la séquence nucléotidique SEQ ID NO : 1.

La séquence de la GFAT2 humaine est notamment décrite dans Oki et al., Genomics (1999) 57:227, et correspond à la séquence nucléotidique SEQ ID NO : 3.

La séquence de la GFAT1Alt humaine est notamment décrite dans DeHaven et al., Diabetes (2001) 50:2419, et correspond à la séquence nucléotidique SEQ ID NO : 5.

L'invention concerne en particulier une protéine ci-dessus, dans laquelle la séquence de l'étiquette de purification est insérée entre deux acides aminés consécutifs, lesdits acides aminés étant compris entre les acides aminés:
- 43 à 47 et/ou 298 à 306, de SEQ ID NO : 2,
- 42 à 45 et/ou 299 à 307, de SEO ID NO :4
- 42 à 45 et/ou 299 à 307, de SEQ ID NO : 4
- 43 à 47 et/ou 316 à 324, de SEQ ID NO: 6

Les acides aminés 43 à 47 de SEQ ID NO : 2, 42 à 45 de SEQ ID NO : 4 et 43 à 47 de SEQ ID NO : 6 correspondent, à savoir sont homologues, à la partie de la séquence de la GFAT d'*E*. *coli* s'étendant entre le feuillet β2 et le feuillet β3, c'est-à-dire à la séquence s'étendant approximativement entre les acides aminés 30 à 80 de la GFAT d'E. *coli.*

Les acides aminés 298 à 306 de SEQ ID NO : 2, 299 à 307 de SEQ ID NO : 4 et 325 à 330 de SEQ ID NO : 6 correspondent, à savoir sont homologues, à la partie de la séquence de la GFAT d'*E*. *coli* s'étendant entre le feuillet β13 et le feuillet β14, c'est-à-dire à la séquence s'étendant approximativement entre les acides aminés 220 à 230 de la GFAT d'E. *coli.*

Selon un autre mode de réalisation particulier, l'invention concerne plus particulièrement une protéine telle que définie ci-dessus, dans laquelle la séquence de l'étiquette de purification est insérée entre deux acides aminés consécutifs de la séquence de GFAT, lesdits acides aminés étant compris dans :
- une partie de la séquence de la GFAT correspondant et/ou étant homologue à la séquence s'étendant approximativement entre les acides aminés 43 à 47 de la GFAT1 humaine,
- une partie de la séquence de la GFAT correspondant et/ou étant homologue à la séquence s'étendant approximativement entre les acides aminés 298 à 306, notamment 299 à 300, de la GFAT1 humaine,

L'invention concerne plus particulièrement une protéine ci-dessus, dans laquelle la séquence de l'étiquette de purification est insérée entre les acides aminés :
- 299 et 300 de SEQ ID NO : 2,
- 300 et 301 de SEQ ID NO : 4,
- 317 et 318 de SEQ ID NO: 6.

L'invention concerne notamment une protéine ci-dessus, dans laquelle l'étiquette de purification correspond à une séquence d'environ 2 à environ 10 acides aminés, notamment d'environ 4 à environ 8 acides aminés.

Des étiquettes de purification préférées selon l'invention concernent notamment des étiquettes dites FLAG (Sigma-Aldrich, France). Ces étiquettes se lient spécifiquement à un paratope donné, ledit paratope pouvant appartenir à un anticorps ou à un fragment d'anticorps par exemple. Un exemple particulier d'étiquette FLAG est constitué par la séquence peptidique Asp-Tyr-Lys-Asp-Asp-Asp-Asp-Asp-Lys (SEQ ID NO : 18) par exemple.

D'autres étiquettes préférées selon l'invention sont des étiquettes formées de plusieurs histidines. Ces étiquettes peuvent former des complexes avec des cations métalliques divalents tels que Ni²⁺ ou Co²⁺ par exemple.

L'invention concerne en particulier une protéine telle que définie ci-dessus, dans laquelle l'étiquette de purification est une hexa-histidine.

On désigne par hexa-histdine la séquence His-His-His-His-His-His (SEQ ID NO: 19).

L'invention concerne plus particulièrement une protéine telle que définie ci-dessus correspondant aux séquences :
- SEQ ID NO : 8, correspondant à la séquence SEQ ID NO : 2 dans laquelle une hexa-histidine est insérée entre les acides aminés 299 et 300,
- SEQ ID NO : 10, correspondant à la séquence SEQ ID NO : 4 dans laquelle une hexa-histidine est insérée entre les acides aminés 300 et 301, et
- SEQ ID NO : 12, correspondant à la séquence SEQ ID NO: 6 dans laquelle une hexa-histidine est insérée entre les acides aminés 317 et 318.

La présente invention concerne également un acide nucléique comprenant ou étant constitué d'une séquence codant pour une protéine telle que définie ci-dessus.

L'invention concerne plus particulièrement un acide nucléique comprenant ou étant constitué de la séquence nucléotidique :
- SEQ ID NO : 7 codant pour la protéine SEQ ID NO : 8, ou
- SEQ ID NO : 9 codant pour la protéine. SEQ ID NO : 10, ou
- SEQ ID NO : 11 codant pour la protéine SEQ ID NO : 12,
ou de son complémentaire, ou étant dérivé de ladite séquence par mutation, insertion ou délétion d'au moins un nucléotide, sous réserve que ladite séquence nucléotidique code pour une protéine enzymatiquement active.

Selon un autre mode de réalisation, la présente invention concerne également un vecteur eucaryote ou procaryote comprenant un acide nucléique tel que défini ci-dessus.

Ces vecteurs permettent notamment de synthétiser les protéines selon l'invention dans un organisme eucaryote ou procaryote.

Avantageusement l'invention concerne un vecteur d'expression de type baculovirus permettant la synthèse des protéines selon l'invention dans des cellules d'insecte.

La présente invention concerne également un procédé de purification d'une protéine telle que définie ci-dessus, à partir d'une solution comprenant ladite protéine, comprenant une étape de mise en présence de ladite solution avec un composé se liant spécifiquement à l'étiquette de purification de ladite protéine et une étape de séparation du complexe formé par la liaison de ladite protéine audit composé des autres constituants de la solution.

Le composé peut être fixé sur un support solide de sorte que le complexe formé entre ledit composé et ladite protéine peut être récupéré par centrifugation ou filtration. Optionnellement ledit composé fixé sur son support peut être disposé dans une colonne à travers laquelle ladite solution est éluée.

Avantageusement, le procédé ci-dessus peut également comprendre une étape de dissociation du complexe formé par la liaison de ladite protéine audit composé afin de récupérer la protéine purifiée.

L'invention concerne plus particulièrement un procédé de purification tel que défini ci-dessus, comprenant une étape de mise en présence d'une solution comprenant une protéine telle que définie ci-dessus avec un composé comprenant un cation métallique divalent choisi parmi le Ni²⁺ ou le Co²⁺, notamment le Ni²⁺, et une étape de séparation du complexe formé par la liaison de la protéine audit composé des autres constituants de la solution.

Avantageusement, le procédé ci-dessus peut également comprendre une étape de dissociation du complexe formé par la liaison de ladite protéine audit composé comprenant un cation métallique divalent, notamment à l'aide d'imidazole, afin de récupérer la protéine purifiée.

Selon un autre mode de réalisation la présente invention concerne un procédé de conservation d'une protéine telle que définie ci-dessus sous une forme enzymatiquement active, notamment à -80°C ou à 4°C, comprenant l'adjonction de ladite protéine à une solution comprenant :
- d'environ 1 mM à environ 10 mM de fructose 6-phosphate, notamment environ 1 mM,
- d'environ 1 mM à environ 5 mM de Tris(2-carboxyethyl)phosphine, notamment environ 1 mM,
- d'environ 5 % à environ 20 % de glycérol, notamment environ 10%.

Le fructose-6-phosphate est un substrat de ladite protéine.

Le Tris(2-carboxyethyl)phosphine est un composé réducteur permettant avantageusement de maintenir les propriété de résines portant des ions Ni²⁺ ou Co²⁺.

Avantageusement le glycérol est un agent cryoprotectant.

A ce titre la présente invention concerne également une composition comprenant une protéine GFAT active liée à une étiquette de purification telle qu'une protéine telle que définie ci-dessus, ladite protéine étant susceptible de pouvoir être conservée sous une forme enzymatiquement active, durant au moins 8 jours à une température de 2°C à 10°C, notamment environ 4°C, et durant au moins 12 mois à une température de - 100°C à -20°C, notamment environ -80°C, ladite protéine étant en association avec :
- environ 1 mM à environ 10 mM de fructose 6-phosphate, notamment environ 1 mM,
- environ 1 mM à environ 5 mM de Tris(2-carboxyethyl)phosphine, notamment environ 1 mM,
- environ 5 % à environ 20 % de glycérol, notamment environ 10%.

La présente invention concerne également l'utilisation d'une protéine telle que définie ci-dessus, pour le criblage de composés modifiant l'activité de ladite protéine, en particulier pour le criblage d'inhibiteur de ladite protéine.

L'activité des protéines selon l'invention peut être en particulier mesurée à l'aide des méthodes suivantes :
- la méthode radiométrique décrite par Broschat et al., Analytical Biochem. (2002) 305:10-15,
- la méthode dite au Nitro Bleu Tétrazolium décrite par Nakata et al., J. Antibio. (2001) 54:737-743.
- la méthode Morgan-Elson décrite par Ghosh et al., Method. Enzymol. (1960) 5:414 et détaillée dans l'exemple qui suit.
- la méthode APAD décrite par Badet et al., Biochemistry (1987) 26:1940 et détaillée dans l'exemple qui suit.

Avantageusement ces méthodes peuvent être utilisées pour le criblage, notamment à haut débit, de composés modifiant l'activité des protéines selon l'invention.

L'invention concerne en particulier l'utilisation telle que définie ci-dessus, pour le criblage de composés utiles dans le cadre du traitement ou de la prévention du diabète, notamment du diabète de type II, de l'obésité, de l'acidose, de la cétose, de l'arthrite, des cancers, ou de l'ostéoporose.

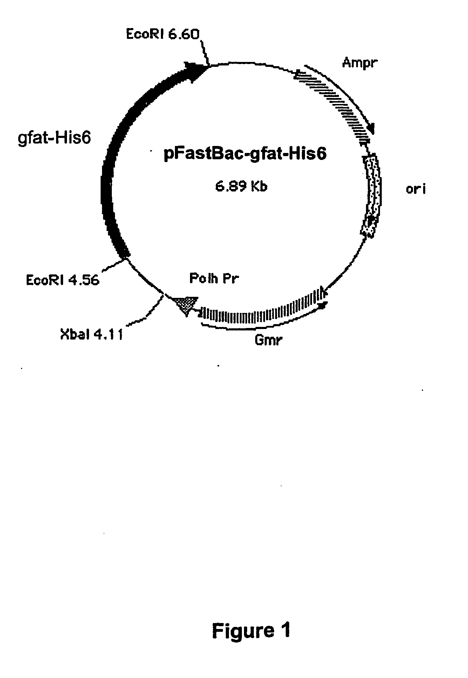

### DESCRIPTION DE LA FIGURE 1

La Figure 1 représente le plasmide pFastBac-gfat-His6 de poids moléculaire 6,89 kb. La cassette « Ampr » représente un gène de résistance à l'ampicilline, la cassette « ori » représente une origine de réplication bactérienne, la cassette « Gmr » représente un gène de résistance à la gentamicine, la cassette « Polh Pr » représente le promoteur de la polyhédrine, la cassette gfat-his6 représente le gène gfat1 modifié par l'insertion d'une séquence codant pour une étiquette hexahistidine. Les sites de restriction *Xba*I en position 4,11 kb, et *Eco*RI en positions 4,56 kb et 6,60 kb sont également représentés.

### EXEMPLE 1

### 1. Synthèse et clonage du gène gfat1-His6

Le fragment *Eco*RI d'un ADNc correspondant au gène *gfat1* humain a été cloné dans le site *Eco*R I du vecteur pCRII (Invitrogen) pour former le plasmide pCRII-gfat1. La séquence nucléotidique d'une étiquette de purification interne composée de 6 résidus histidine a été introduite en position 898 de la séquence du gène *gfat1* cloné dans pCRII par PCR avec la *Platinum pfx polymerase* (Roche) et la paire d'amorces appropriées :
- Start Aat II-His6 :
- End Hinc II :
   5' CAAAGTTGACTCTTCCTCTCATTGTGTTCACGACAGACTCTGGC 3' (SEQ ID NO : 15)
selon le protocole suivant : 94°C, 2min puis 30 cycles (94°C 45 sec, 55°C 1 min, 72°C 5 min) suivis d'une polymérisation de 5 min à 72°C et retour à 4°C.

Après digestion par *Aat*II et *Hinc*II puis purification sur gel d'agarose Seaplaque 1,5% (Tebu), l'amplicon (170 bp) a été inséré au niveau des sites de restriction correspondants dans la construction pCRII-gfat1. L'insert de 170 pb a été introduit par ligation dans la construction avec un rapport 3:1 à 16°C pendant la nuit en présence de T4 DNA ligase (Nebs). Le mélange de ligation (20 µl) ainsi obtenu a permis de transformer une souche d'*E*. *coli* JM109. Ensuite, le fragment *Xba*I*- Hind*III du plasmide recombinant pCRII-gfatl-His6 a été cloné dans le plasmide donneur pFastBac1 (Life Technologies Ltd). Le plasmide pFastBac-gfat-His6 ainsi généré (**Figure 1**) a été vérifié par digestions multiples: *Sma*I*, Acc*I/*Dra*I, *PstE*I*lXba*I*,* et par séquençage. En vue d'améliorer la construction, la séquence en amont du codon de démarrage a été mutée sur deux positions par PCR, avec la paire d'amorces suivante, afin d'enlever deux phases de lecture ouvertes en amont du gène *gfat1:*
- Start XbaI
   5' AATCTAGATTCATGCTCGAGCGGCCGCCAGTGTGATTGATATC 3' (SEQ ID NO : 16)
- End AfeI
   5' ATTTTTATCAGAGCGCTGGGGGTGGCTATTGACAGG 3' (SEQ ID NO : 17)
selon le protocole : 94°C 2 min, puis 30 cycles (94°C 15 sec, 55°C 30 sec, 68°C 1 min) suivis d'une polymérisation de 1 min à 68°C et retour à 4°C.

Le fragment de PCR obtenu, contenant les deux mutations, a été purifié sur gel SeaPlaque (Tebu) à 0,7% puis digéré par *Xba*I et *Afe*I pour remplacer son homologue dans pFastBac-gfat-His6 afin de donner le plasmide donneur pFastBac-gfat-His6-2orf servant à la transposition dans les cellules DH10Bac (Life Technologies Ltd). La construction a été vérifiée par digestions *Sma*I, *XbaI*/*Pst*EI*, Xba*I/*Hind*III, et par séquençage.

Un bacmide recombinant a été isolé après transposition dans les cellules DH10Bac et utilisé pour transfecter des cellules d'insecte Sf9 en présence de Lipofectin (Life Technologies Ltd). Les baculovirus obtenus ont été amplifiés dans les cellules Sf9 et le titre viral a été mesuré à 5.10⁷ pfu/ml.

### 2. Production de la protéine GFAT1-His6

Des cellules d'insectes Sf9 ont été cultivées à 28°C en présence de milieu SF900II (Life Technologies Ltd) en fioles de 5 L sous agitation à 100 rpm. Les cellules à une densité de 2.10⁹ cellules /L ont été infectées par le baculovirus recombinant obtenu ci-dessus avec une multiplicité d'infection de 2 (pfu/cellule), puis cultivées durant 72 h.

Les cellules et le surnageant ont été séparés par centrifugation (2500 g, 10 min à 4°C). Les culots cellulaires ont été lavés en présence de tampon Tris-HCl 20 mM, pH 7, centrifugés (4000 g, 45 min à 4°C) et congelés à -80°C.

### 3. Purification de la protéine GFAT1-His6

Le culot cellulaire (20 g) a été repris dans 50 ml de tampon de lyse (NaPO₄ 50mM pH 7,5, NaCl 300 mM, imidazole 10 mM, fructose-6-phosphate (fructose-6P) 1 mM, TCEP (Tris(2-carboxyethyl)phosphine) 1 mM, PMSF 1 mM (fluorure de phénylmethylsulfonyl), 10% glycérol et 1 tablette de cocktail d'inhibiteurs de protéases sans EDTA (Roche Applied Sciences) et soumis à un broyage au DynoMill à 4500 trs/min (4 cycles de 30 sec) en présence de 40 g de microbilles de 0,2 mm de diamètre. L'ensemble a été refroidi par circulation d'ethylène glycol/eau réglé à -15°C. L'extrait brut obtenu (100 ml, 445 mg de protéines totales) a été centrifugé à 4°C pendant 20 min à 12000 tpm. Le surnageant a été soumis à une ultracentrifugation à 4°C (350000 tpm, 1h). Le surnageant ainsi obtenu a été mélangé à 5 ml de matrice 50% Ni-NTA (Qiagen) pendant 2 h à 4°C. Le mélange a été coulé dans une colonne vide puis rincé avec 40 ml de tampon de lavage (NaPO₄ 50 mM pH 7,5, NaCl 300 mM, imidazole 40 mM, fructose-6P 1 mM, TCEP 1 mM, PMSF 1 mM et 1 tablette de cocktail d'inhibiteurs de protéases sans EDTA (Roche Applied Sciences). L'élution a été réalisée par paliers successifs à 125 et 500 mM imidazole dans le même tampon que précédemment. 12 mg de GFAT1-His6 fonctionnelle (dosage protéique selon la méthode de Bradford ont ainsi été obtenus.

### 4. Conservation de l'enzyme GFAT1-His6

L'enzyme a alors été stockée en fractions de 100 µl en présence de 1 mM fructose-6P, 1mM TCEP et 10% glycérol à -80°C. La stabilité de l'enzyme est de plusieurs mois à -80°C et supérieure à 8 jours à 4°C.

### 5. Dosage de l'activité de l'enzyme GFAT1-His6

Différents tests de dosage de l'activité enzymatique de la GFAT1-His6 ont été utilisés. Ces tests peuvent être également utilisés afin de cribler des composés modifiant, et notamment inhibant, l'activité de la GFAT1-His6. Il est possible des les adapter aisément à un criblage à haut débit.

### Dosage de Morgan-Elson :

Dans ce cas l'activité enzymatique est suivie par un test colorimétrique dont le principe est le suivant : la D-glucosamine-6P libérée par l'enzyme est N-acétylée par l'anhydride acétique en milieu alcalin (Ghosh et al., Method Enzymol. (1962) 5:414), puis La solution est traitée par le réactif d'Ehrlich (para-diméthyl-amino-benzaldéhyde, PDAB) en milieu acide concentré ; le composé rose formé absorbe à 585 nm.

La réaction enzymatique se déroule pendant 30 min à 37°C en présence de :
- 0,2 ml de fructose-6P à 100 mM
- 0,25 ml de L-Glutamine à 60 mM
- 0,25 ml de tampon KPO₄ à 150 mM pH 7
- 0,1 ml d'EDTA (éthylène diamine tétra-acétate) à 25 mM, pH 7
- jusqu'à 200 µl d'échantillon (à compléter avec H₂O si nécessaire)

La réaction est stoppée par immersion 4 minutes dans un bain d'eau à 100°C puis centrifugée. 0,8 ml du surnageant sont prélevés pour le dosage de la glucosamine-6P suivant le protocole suivant :
- addition de 0,1 ml de NaHCO₃ saturé,
- addition de 0,1 ml d'une solution d'anhydride acétique à 5 % dans l'eau préparée extemporanément,
- agitation et incubation 5 min à température ambiante,
- incubation 5 min dans un bain à 100°C,
- addition de 0,2 ml de borate de potassium 0,8 M pH 9,1 (à ajuster avec KOH 10 N).
- agitation et incubation 7 min dans un bain à 100°C.
- addition de 3 ml de réactif d'Ehrlich dilué 10 fois dans l'acide acétique, préparé extemporanément, sur la solution refroidie dans la glace,
- incubation 20 min à 37°C.

L'activité de la GFAT à été déterminée comparativement à une courbe étalon établie en utilisant la D-glucosamine comme standard dans une gamme de concentration de 0 à 200 nmoles. L'activité spécifique de la GFAT1-His6 obtenue a été ainsi mesurée à 1,7 U/mg. Ce qui est supérieur à la valeur de 0,4 U/mg obtenue par Broschat et al., J. Biol. Chem. (2002) 277:14764, pour la purification d'une GFAT1 humaine recombinante. Ceci traduit une activité supérieure de la GFAT1-His6 et/on une pureté plus importante de la préparation enzymatique selon l'invention.

Les paramètres cinétiques de la GFAT1-His6 ont été caractérisés vis-à-vis de la glutamine (Kₘ^{Gln} = 0,2 mM) et du fructose-6P (F6P) (Kₘ^{F6P} = 0,006 mM) par un dosage spectrophotométrique couplé à la glutamate déshydrogénase selon le test APAD. Ce qui est conforme aux valeurs citées dans l'art antérieur (Kₘ^{Gln} = 0,26 mM et Kₘ^{F6P} = 0,007 mM pour Broschat et al., J. Biol. Chem. (2002) 277:14764).

### Dosage APAD

Il s'agit d'un dosage spectrophotométrique dans l'ultraviolet de l'activité GFAT. Il est basé sur la détermination, en continu, de la quantité de L-glutamate formé à l'aide de la GFAT et d'un analogue du NAD (nicotinamide adénine dinucléotide), l'APAD (3-acetylpyridine adénine dinucleotide), selon la réaction suivante (catalysée par la glutamate déshydrogénase (GDH)):

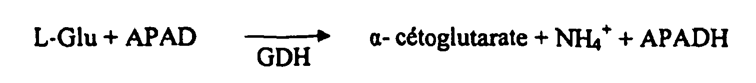

La mesure s'effectue à 365 nm, à 37°C. Dans ces conditions une unité d'absorbance correspond à 0,11 µmole d'APADH formé.

L'essai comprend :
- 100 µl APAD 3 mM (2 mg/ml)
- 25 µl KCl 2M
- 100 µl de tampon KPO₄ 1 M pH 7,2
- 100 µl de Fructose-6P 100 mM (30,41 mg/ml)
- 100 µl de L-Glutamine 60 mM purifiée (8,77 mg/ml)
- H₂O qsp 1 ml (en tenant compte des volumes à rajouter ci-après)
- 50 µl GDH
- échantillon à doser : 0,5 µg

Il est également possible d'utiliser d'autres procédés de dosage, tels que le dosage radiométrique décrit par Broschat et al., Analytical Biochem. (2002) 305:10-15 ou le dosage dit Nitro Bleu Tétrazolium décrit par Nakata et al., J. Antibio. (2001) 54:737-743.

## Revendications

1. Protéine possédant une activité GFAT comprenant :
une séquence de GFAT et au moins une séquence d'une étiquette de purification, la séquence de l'étiquette de purification étant insérée entre deux acide aminés consécutifs
de la séquence de GFAT, lesdits acides aminés étant compris dans :
• une séquence de GFAT correspondant à la séquence s'étendant entre les acides aminés 30 à 80 de la GFAT d'Escherichia coli, ou
• une séquence de GFAT correspondant à la séquence s'étendant entre les acides aminés 220 à 230 de la GFAT d'Escherichia coli.

2. Protéine selon la revendication 1, dans laquelle la séquence de GFAT correspond à une séquence de GFAT de bactérie ou d'eucaryote, notamment de plante, de champignon ou d'animal, en particulier d'insecte ou de mammifère, plus particulièrement d'homme.

3. Protéine selon la revendication 1 ou 2, dans laquelle la séquence de l'étiquette de purification est insérée entre deux acides aminés consécutifs de la séquence d'un GFAT humaine, lesdits acides aminés étant compris entre les acides aminés 40 à 50 et/ou 290 à 330 de ladite séquence de GFAT humaine.

4. Protéine selon l'une des revendications 1 à 3, dans laquelle la séquence de GFAT correspond à :
• SEQ ID NO : 2, correspondant à la séquence de la GFAT1 humaine, ou
• SEQ ID NO : 4, correspondant à la séquence de la GFAT2 humaine, ou
• SEQ ID NO: 6, correspondant à la séquence de la GFAT1Alt humaine.

5. Protéine selon l'une des revendications 1 à 4, dans laquelle la séquence de l'étiquette de purification est insérée entre deux acides aminés consécutifs, lesdits acides aminés étant compris entre les acides aminés :
• 43 à 47 et/ou 298 à 306 de SEQ ID NO : 2, ou
• 42 à 45 et/ou 299 à 307 de SEQ ID NO : 4, ou
• 43 à 47 et/ou 316 à 324 de SEQ ID NO : 6.

6. Protéine selon la revendication 5, dans laquelle la séquence de l'étiquette de purification est insérée entre les acides aminés :
• 299 et 300 de SEQ 10 NO : 2, ou
• 300 et 301 de SEQ ID NO : 4, ou
• 317 et 318 de SEQ ID NO : 6

7. Protéine selon l'une des revendications 1 à 6, dans laquelle l'étiquette de purification correspond à une séquence de 2 à 10 acides aminés, notamment de 4 à 8 acides aminés.

8. Protéine selon l'une des revendications 1 à 7, dans laquelle l'étiquette de purification est une hexa-histidine.

9. Protéine selon les revendications 6 et 8 correspondant aux séquences :
• SEQ ID NO : 8, correspondant à la séquence SEQ ID NO : 2 dans laquelle une hexa-histidine est insérée entre les acides aminés 299 et 300,
• SEQ ID NO : 10, correspondant à la séquence SEQ ID NO : 4 dans laquelle une hexa-histidine est insérée entre les acides aminés 300 et 301, et
• SEQ ID NO : 12, correspondant à la séquence SEQ ID NO : 6 dans laquelle une hexa-histidine est insérée entre les acides aminés 317 et 318.

10. Acide nucléique comprenant ou étant constitué d'une séquence codant pour une protéine selon l'une des revendications 1 à 9.

11. Acide nucléique comprenant ou étant constitué de la séquence nucléotidique :
• SEQ ID NO : 7 codant pour la protéine SEQ ID NO : 8, ou
• SEQ IN NO : 9 codant pour la protéine SEQ ID NO : 10, ou
• SEQ ID NO : 11 codant pour la protéine SEQ ID NO : 12.

12. Vecteur eucaryote ou procaryote comprenant un acide nucléique selon la revendication 10 ou 11.

13. Procédé de purification d'une protéine selon l'une des revendications 1 à 9 , à partir d'une solution comprenant ladite protéine, comprenant une étape de mise en présence de ladite solution avec un composé se liant spécifiquement à l'étiquette de purification de ladite protéine et une étape de séparation du complexe formé par la liaison de ladite protéine audit composé des autres constituants de la solution.

14. Procédé de purification selon la revendication 13 , comprenant une étape de mise en présence d'une solution comprenant une protéine selon la revendication 9 avec un composé comprenant un cation métallique divalent choisi parmi le Ni²⁺ ou le Co²⁺, notamment le Ni²⁺, et une étape de séparation du complexe formé par la liaison de la protéine audit composé des autres constituants de la solution.

15. Procédé de conservation d'une protéine selon l'une des revendications 1 à 9 sous une forme enzymatiquement active, notamment à -80°C ou à 4°C, comprenant l'adjonction de ladite protéine à une solution comprenant :
• de 1 mM à 10 mM de fructose 6-phosphate, notamment environ 1 mM,
• de 1 mM à 5 mM de Tris(2-carboxyethyl)phosphine, notamment environ 1 mM,
• de 5 % à 20 % de glycérol, notamment environ 10%.

16. Composition comprenant une protéine GFAT active liée à une étiquette de purification selon l'une des revendications 1 à 9, ladite protéine étant susceptible de pouvoir être conservée sous une forme enzymatiquement active, durant au moins 8 jours à une température de 2°C à 10°C, notamment 4°C, et durant au moins 12 mois à une température de -100°C à -20°C, notamment -80°C, ladite protéine étant en association avec :
• 1 mM à 10 mM de fructose 6-phosphate, notamment 1 mM,
• 1 mM à 5 mM de Tris(2-carboxyethyl)phosphine, notamment 1 mM,
• 5 % à 20 % de glycérol, notamment 10%.

17. Utilisation d'une protéine selon l'une des revendications 1 à 9, pour le criblage de composés modifiant l'activité de ladite protéine, en particulier pour le criblage d'inhibiteur de ladite protéine.

18. Utilisation selon la revendication 17, pour le criblage de composés utiles dans le cadre du traitement ou de la prévention du diabète, notamment du diabète de type II, de l'obésité, de l'acidose, de la cétose, de l'arthrite, des cancers, ou de l'ostéoporose.

## Claims

1. Protein possessing a GFAT activity comprising:
a GFAT sequence and at least one purification tag sequence, the purification tag sequence being inserted between two consecutive amino acids of the GFAT sequence, said amino acids being comprised in
- a GFAT sequence corresponding to the sequence extending between amino acids 30 to 80 of the *Escherichia coli* GFAT, or
- a GFAT sequence corresponding to the sequence extending between amino acids 220 to 230 of the *Escherichia coli* GFAT.

2. Protein according to claim 1, in which the GFAT sequence corresponds to a bacterial or eukaryotic, in particular plant, fungal or animal, in particular insect or mammal, more particularly human GFAT sequence.

3. Protein according to claim 1 or 2, in which the purification tag sequence is inserted between two consecutive amino acids of a human GFAT sequence, said amino acids being included between amino acids 40 to 50 and/or 290 to 330 of said human GFAT sequence.

4. Protein according to anyone of claims 1 to 3, in which the GFAT sequence corresponds to:
- SEQ ID NO: 2, corresponding to the human GFAT1 sequence, or
- SEQ ID NO: 4, corresponding to the human GFAT2 sequence, or
- SEQ ID NO: 6, corresponding to the human GFAT I Alt sequence.

5. Protein according to anyone of claims 1 to 4, in which the purification tag sequence is inserted between two consecutive amino acids, said amino acids being included between amino acids:
- 43 to 47 and/or 298 to 306 of SEQ ID NO: 2, or
- 42 to 45 and/or 299 to 307 of SEQ ID NO: 4, or
- 43 to 47 and/or 316 to 324 of SEQ ID NO: 6.

6. Protein according to claim 5, in which the purification tag sequence is inserted between amino acids:
- 299 and 300 of SEQ ID NO: 2, or
- 300 and 301 of SEQ ID NO: 4, or
- 317 and 318 of SEQ ID NO: 6.

7. Protein according to anyone of claims 1 to 6, in which the purification tag corresponds to a sequence of 2 to 10 amino acids, in particular 4 to 8 amino acids.

8. Protein according to anyone of claims 1 to 7, in which the purification tag is a hexa-histidine.

9. Protein according to claims 6 and 8, corresponding to the sequences:
- SEQ ID NO: 8, corresponding to the sequence SEQ ID NO: 2 in which a hexahistidine is inserted between amino acids 299 and 300,
- SEQ ID NO: 10, corresponding to the sequence SEQ ID NO: 4 in which a hexahistidine is inserted between amino acids 300 and 301, and
- SEQ ID NO: 12, corresponding to the sequence SEQ ID NO: 6 in which a hexahistidine is inserted between amino acids 317 and 318.

10. Nucleic acid comprising or being constituted by a sequence coding for a protein according one of the claims 1 to 9.

11. Nucleic acid comprising or being constituted by the nucleotide sequence:
- SEQ ID NO: 7 coding for the protein SEQ ID NO: 8, or
- SEQ ID NO: 9 coding for the protein SEQ ID NO: 10, or
- SEQ ID NO: 11 coding for the protein SEQ ID NO: 12.

12. A eukaryotic or prokaryotic vector comprising a nucleic acid of claim 10 or 11.

13. Purification process for a protein according one of the claims 1 to 9, from a solution comprising said protein, comprising a stage of bringing said solution into the presence of a compound binding specifically to the purification tag of said protein and a stage of separation of the complex formed by the binding of said protein to said compound from the other constituents of the solution.

14. Purification process of claim 13, comprising a stage of bringing a solution comprising a protein according to claim 9, into the presence of a compound comprising a divalent metallic cation chosen among Ni²⁺ or Co²⁺, in particular Ni²⁺, and a stage of separation of the complex formed by the binding of the protein to said compound from the other constituents of the solution.

15. Conservation process for a protein according one of the claims 1 to 9 in an enzymatically-active form, in particular at -80°C or at 4°C, comprising the addition of said protein to a solution comprising:
- 1 mM to 10 mM of fructose 6-phosphate, in particular approximately 1 mM,
- 1 mM to 5 mM of Tris(2-carboxyethyl)phosphine, in particular environ 1 mM,
- 5% to 20% of glycerol, in particular approximately 10%.

16. Composition comprising an active GFAT protein, bound to a purification tag, according one of the claims 1 to 9, said protein being capable of being preserved in an enzymatically-active form, for at least 8 days at a temperature of 2°C to 10°C, in particular 4°C, and for at least 12 months at a temperature of -100°C to -20°C, in particular -80°C, said protein being in combination with:
- 1 mM to 10 mM of fructose 6-phosphate, in particular 1 mM,
- 1 mM to 5 mM of Tris(2-carboxyethyl)phosphine, in particular 1 mM,
- 5% to 20% of glycerol, in particular 10%.

17. Use of a protein according one of the claims 1 to 9, for the screening of compounds modifying the activity of said protein, in particular for the screening inhibitor of said protein.

18. Use of a protein according to claim 17, for the screening of compounds useful within the framework of the treatment or prevention of diabetes, in particular type II diabetes, obesity, acidosis, ketosis, arthritis, cancer, or osteoporosis.

## Patentansprüche

1. Protein, das eine GFAT-Aktivität besitzt, umfassend:
eine GFAT-Sequenz und mindestens eine Reinigungs-Tag-Sequenz, wobei die Reinigungs-Tag-Sequenz zwischen zwei aufeinanderfolgenden Aminosäuren der GFAT-Sequenz eingefügt ist, wobei die Aminosäuren enthalten sind in:
• einer GFAT-Sequenz, die der Sequenz entspricht, die sich zwischen den Aminosäuren 30 bis 80 der GFAT von Eschericia coli erstreckt, oder
• einer GFAT-Sequenz, die der Sequenz entspricht, die sich zwischen den Aminosäuren 220 bis 230 der GFAT von Eschericia coli erstreckt.

2. Protein nach Anspruch 1, wobei die GFAT-Sequenz einer GFAT-Sequenz eines Bakteriums oder Eukaryoten, insbesondere einer Pflanze, eines Pilzes oder eines Tiers, besonders einem Insekt oder einem Säuger, ganz speziell dem Menschen entspricht.

3. Protein nach Anspruch 1 oder 2, wobei die Reinigungs-Tag-Sequenz zwischen zwei aufeinanderfolgenden Aminosäuren der Sequenz einer humanen GFAT eingefügt ist, wobei die Aminosäuren zwischen den Aminosäuren 40 bis 50 und/oder 290 bis 330 der humanen GFAT-Sequenz enthalten sind.

4. Protein nach einem der Ansprüche 1 bis 3, wobei die GFAT-Sequenz Folgendem entspricht:
• SEQ ID NR: 2, die der humanen GFAT1-Sequenz entspricht,oder
• SEQ ID NR: 4, die der humanen GFAT2-Sequenz entspricht, oder
• SEQ ID NR: 6, die der humanen GFAT1Alt-Sequenz entspricht.

5. Protein nach einem der Ansprüche 1 bis 4, wobei die Reinigungs-Tag-Sequenz zwischen zwei aufeinanderfolgenden Aminosäuren eingefügt ist, wobei die Aminosäuren zwischen den folgenden Aminosäuren enthalten sind:
• 43 bis 47 und/oder 298 bis 306 der SEQ ID NR: 2, oder
• 42 bis 45 und/oder 299 bis 307 der SEQ ID NR: 4, oder
• 43 bis 47 und/oder 316 bis 324 der SEQ ID NR: 6.

6. Protein nach Anspruch 5, wobei die Reinigungs-Tag-Sequenz zwischen die folgenden Aminosäuren eingefügt ist:
• 299 und 300 von SEQ ID NR: 2, oder
• 300 und 301 von SEQ ID NR: 4, oder
• 317 und 318 von SEQ ID NR: 6.

7. Protein nach einem der Ansprüche 1 bis 6, wobei der Reinigungs-Tag einer Sequenz mit 2 bis 10 Aminosäuren, insbesondere 4 bis 8 Aminosäuren entspricht.

8. Protein nach einem der Ansprüche 1 bis 7, wobei der Reinigungs-Tag ein Hexahistidin ist.

9. Protein nach den Ansprüchen 6 und 8, das den folgenden Sequenzen entspricht:
• SEQ ID NR: 8, die der Sequenz SEQ ID NR: 2 entspricht, in die ein Hexahistidin zwischen die Aminosäuren 299 und 300 eingefügt ist,
• SEQ ID NR: 10, die der Sequenz SEQ ID NR: 4 entspricht, in die ein Hexahistidin zwischen die Aminosäuren 300 und 301 eingefügt ist, und
• SEQ ID NR: 12, die der Sequenz SEQ ID NR: 6 entspricht, in die ein Hexahistidin zwischen die Aminosäuren 317 und 318 eingefügt ist.

10. Nukleinsäure, umfassend oder bestehend aus einer Sequenz, die für ein Protein nach einem der Ansprüche 1 bis 9 kodiert.

11. Nukleinsäure, umfassend oder bestehend aus der Nukleotidsequenz:
• SEQ ID NR: 7, die für das Protein SEQ ID NR: 8 kodiert, oder
• SEQ ID NR: 9, die für das Protein SEQ ID NR: 10 kodiert, oder
• SEQ ID NR: 11, die für das Protein SEQ ID NR: 12 kodiert.

12. Eukaryotischer oder prokaryotischer Vektor, der eine Nukleinsäure nach Anspruch 10 oder 11 umfasst.

13. Verfahren zur Reinigung eines Proteins nach einem der Ansprüche 1 bis 9, ausgehend von einer Lösung, die das Protein umfasst, das einen Schritt des Inkontaktbringens der Lösung mit einer Verbindung umfasst, die spezifisch an das Reinigungs-Tag des Proteins bindet und einen Schritt der Trennung des Komplexes, der durch die Bindung des Proteins an die Verbindung gebildet wurde, von den anderen Bestandteilen der Lösung.

14. Verfahren zur Reinigung nach Anspruch 13, das einen Schritt des Inkontaktbringens einer Lösung, die ein Protein nach Anspruch 9 umfasst, mit einer Verbindung, die ein zweiwertiges Metallkation, wie etwa Ni²⁺ oder Co²⁺, insbesondere Ni²⁺, umfasst, und einen Schritt der Trennung des Komplexes, der durch die Bildung des Proteins an die Verbindung gebildet wurde, von den anderen Bestandteilen der Lösung umfasst.

15. Verfahren zur Konservierung eines Proteins nach einem der Ansprüche 1 bis 9 in enzymatisch aktiver Form, insbesondere bei -80 °C oder bei 4 °C, das die Zugabe des Proteins zu einer Lösung umfasst, die Folgendes umfasst:
• 1 mM bis 10 mM Fructose-6-phosphat, insbesondere etwa 1 mM,
• 1 mM bis 5 mM Tris(2-carboxyethyl)phosphin, insbesondere 1 mM,
• 5 % bis 20 % Glycerol, insbesondere 10 %.

16. Zusammensetzung, die ein aktives GFAT-Protein umfasst, das an einen Reinigungs-Tag nach einem der Ansprüche 1 bis 9 gebunden ist, wobei das Protein in einer enzymatisch aktiven Form mindestens 8 Tage lang bei einer Temperatur von 2 °C bis 10 °C, insbesondere 4 °C, und mindestens 12 Monate lang bei einer Temperatur von -100 °C bis -20 °C, insbesondere - 80 °C, konserviert werden kann, wobei das Protein assoziiert ist mit :
• 1 mM bis 10 mM Fructose-6-phosphat, insbesondere 1 mM,
• 1 mM bis 5 mM Tris(2-carboxyethyl)phosphin, insbesondere 1 mM,
• 5 % bis 20 % Glycerol, insbesondere 10 %.

17. Verwendung eines Proteins nach einem der Ansprüche 1 bis 9 zum Screenen von Verbindungen, die die Aktivität des Proteins modifizieren, besonders zum Screenen des Proteininhibitors.

18. Verwendung nach Anspruch 17 zum Screenen von Verbindungen, die im Rahmen der Behandlung oder der Vorbeugung von Diabetes, insbesondere Typ-II-Diabetes, Adipositas, Acidose, Ketose, Arthritis, Krebsformen oder Osteoporose nützlich sind.


### LISTE DE SEQUENCES

<110> CENTRE NATIONAL DE LA RECHERCHE SCIENTIFIQUE
<120> GLUTAMINE:FRUCTOSE-6-PHOSPHATE AMIDOTRANSFERASE (GFAT) COMPRENANT UNE ETIQUETTE DE PURIFICATION INTERNE, ET SON UTILISATION POUR LE CRIBLAGE DE COMPOSES
<130> WOB 03 BP CNR GFAT
<160> 19
<170> PatentIn version 3.1
<210> 1
   <211> 2046
   <212> ADN
   <213> Homo sapiens
<220>
   <221> CDS
   <222> (1) .. (2046)
   <223>
<220>
   <221> misc_feature
   <222> (170)..(170)
   <223> t ou c
<400> 1
   atg tgt ggt ata ttt gct tac tta aac tac cat gtt cct cga acg aga      48
   Met Cys Gly Ile Phe Ala Tyr Leu Asn Tyr His Val Pro Arg Thr Arg
   1               5                   10                  15
   cga gaa atc ctg gag acc cta atc aaa ggc ctt cag aga ctg gag tac      96
   Arg Glu Ile Leu Glu Thr Leu Ile Lys Gly Leu Gln Arg Leu Glu Tyr
               20                  25                  30
   aga gga tat gat tct gct ggt gtg gga ttt gat gga ggc aat gat aaa     144
   Arg Gly Tyr Asp Ser Ala Gly Val Gly Phe Asp Gly Gly Asn Asp Lys
           35                  40                  45
   gat tgg gaa gcc aat gcc tgc aaa anc cag ctt att aag aag aaa gga     192
   Asp Trp Glu Ala Asn Ala Cys Lys Xaa Gln Leu Ile Lys Lys Lys Gly
       50                  55                  60
   aaa gtt aag gca ctg gat gaa gaa gtt cac aag caa caa gat atg gat     240
   Lys Val Lys Ala Leu Asp Glu Glu Val His Lys Gln Gln Asp Met Asp
   65                  70                  75                  80
   ttg gat ata gaa ttt gat gta cac ctt gga ata gct cat acc cgt tgg     288
   Leu Asp Ile Glu Phe Asp Val His Leu Gly Ile Ala His Thr Arg Trp
                   85                  90                  95
   gca aca cat gga gaa ccc agt cct gtc aat agc cac ccc cag cgc tct     336
   Ala Thr His Gly Glu Pro Ser Pro Val Asn Ser His Pro Gln Arg Ser
               100                 105                 110
   gat aaa aat aat gaa ttt atc gtt att cac aat gga atc atc acc aac     384
   Asp Lys Asn Asn Glu Phe Ile Val Ile His Asn Gly Ile Ile Thr Asn
           115                 120                 125
   tac aaa gac ttg aaa aag ttt ttg gaa agc aaa ggc tat gac ttc gaa     432
   Tyr Lys Asp Leu Lys Lys Phe Leu Glu Ser Lys Gly Tyr Asp Phe Glu
       130                 135                 140
   tct gaa aca gac aca gag aca att gcc aag ctc gtt aag tat atg tat      480
   Ser Glu Thr Asp Thr Glu Thr Ile Ala Lys Leu Val Lys Tyr Met Tyr
   145                 150                 155                 160
   gac aat cgg gaa agt caa gat acc agc ttt act acc ttg gtg gag aga      528
   Asp Asn Arg Glu Ser Gln Asp Thr Ser Phe Thr Thr Leu Val Glu Arg
                   165                 170                 175
   gtt atc caa caa ttg gaa ggt gct ttt gca ctt gtg ttt aaa agt gtt      576
   Val Ile Gln Gln Leu Glu Gly Ala Phe Ala Leu Val Phe Lys Ser Val
               180                 185                 190
   cat ttt ccc ggg caa gca gtt ggc aca agg cga ggt agc cct ctg ttg      624
   His Phe Pro Gly Gln Ala Val Gly Thr Arg Arg Gly Ser Pro Leu Leu
           195                 200                 205
   att ggt gta cgg agt gaa cat aaa ctt tct act gat cac att cct ata      672
   Ile Gly Val Arg Ser Glu His Lys Leu Ser Thr Asp His Ile Pro Ile
       210                 215                 220
   ctc tac aga aca ggc aaa gac aag aaa gga agc tgc aat ctc tct cgt      720
   Leu Tyr Arg Thr Gly Lys Asp Lys Lys Gly Ser Cys Asn Leu Ser Arg
   225                 230                 235                 240
   gtg gac agc aca acc tgc ctt ttc ccg gtg gaa gaa aaa gca gtg gag      768
   Val Asp Ser Thr Thr Cys Leu Phe Pro Val Glu Glu Lys Ala Val Glu
                   245                 250                 255
   tat tac ttt gct tct gat gca agt gct gtc ata gaa cac acc aat cgc      816
   Tyr Tyr Phe Ala Ser Asp Ala Ser Ala Val Ile Glu His Thr Asn Arg
               260                 265                 270
   gtc atc ttt ctg gaa gat gat gat gtt gca gca gta gtg gat gga cgt      864
   Val Ile Phe Leu Glu Asp Asp Asp Val Ala Ala Val Val Asp Gly Arg
           275                 280                 285
   ctt tct atc cat cga att aaa cga act gca gga gat cac ccc gga cga      912
   Leu Ser Ile His Arg Ile Lys Arg Thr Ala Gly Asp His Pro Gly Arg
       290                 295                 300
   gct gtg caa aca ctc cag atg gaa ctc cag cag atc atg aag ggc aac      960
   Ala Val Gln Thr Leu Gln Met Glu Leu Gln Gln Ile Met Lys Gly Asn
   305                 310                 315                 320
   ttc agt tca ttt atg cag aag gaa ata ttt gag cag cca gag tct gtc     1008
   Phe Ser Ser Phe Met Gln Lys Glu Ile Phe Glu Gln Pro Glu Ser Val
                   325                 330                 335
   gtg aac aca atg aga gga aga gtc aac ttt gat gac tat act gtg aat     1056
   Val Asn Thr Met Arg Gly Arg Val Asn Phe Asp Asp Tyr Thr Val Asn
               340                 345                 350
   ttg ggt ggt ttg aag gat cac ata aag gag atc cag aga tgc cgg cgt     1104
   Leu Gly Gly Leu Lys Asp His Ile Lys Glu Ile Gln Arg Cys Arg Arg
           355                 360                 365
   ttg att ctt att gct tgt gga aca agt tac cat gct ggt gta gca aca     1152
   Leu Ile Leu Ile Ala Cys Gly Thr Ser Tyr His Ala Gly Val Ala Thr
       370                 375                 380
   cgt caa gtt ctt gag gag ctg act gag ttg cct gtg atg gtg gaa cta     1200
   Arg Gln Val Leu Glu Glu Leu Thr Glu Leu Pro Val Met Val Glu Leu
   385                 390                 395                 400
   gca agt gac ttc ctg gac aga aac aca cca gtc ttt cga gat gat gtt 1248
   Ala Ser Asp Phe Leu Asp Arg Asn Thr Pro Val Phe Arg Asp Asp Val
   405 410 415
   tgc ttt ttc ctt agt caa tca ggt gag aca gca gat act ttg atg ggt 1296
   Cys Phe Phe Leu Ser Gln Ser Gly Glu Thr Ala Asp Thr Leu Met Gly
   420 425 430
   ctt cgt tac tgt aag gag aga gga gct tta act gtg ggg atc aca aac 1344
   Leu Arg Tyr Cys Lys Glu Arg Gly Ala Leu Thr Val Gly Ile Thr Asn
   435 440 445
   aca gtt ggc agt tcc ata tca cgg gag aca gat tgt gga gtt cat att 1392
   Thr Val Gly Ser Ser Ile Ser Arg Glu Thr Asp Cys Gly Val His Ile
   450 455 460
   aat gct ggt cct gag att ggt gtg gcc agt aca aag gct tat acc agc 1440
   Asn Ala Gly Pro Glu Ile Gly Val Ala Ser Thr Lys Ala Tyr Thr Ser
   465 470 475 480
   cag ttt gta tcc ctt gtg atg ttt gcc ctt atg atg tgt gat gat cgg 1488
   Gln Phe Val Ser Leu Val Met Phe Ala Leu Met Met Cys Asp Asp Arg
   485 490 495
   atc tcc atg caa gaa aga cgc aaa gag atc atg ctt gga ttg aaa cgg 1536
   Ile Ser Met Gln Glu Arg Arg Lys Glu Ile Met Leu Gly Leu Lys Arg
   500 505 510
   ctg cct gat ttg att aag gaa gta ctg agc atg gat gac gaa att cag 1584
   Leu Pro Asp Leu Ile Lys Glu Val Leu Ser Met Asp Asp Glu Ile Gln
   515 520 525
   aaa cta gca aca gaa ctt tat cat cag aag tca gtt ctg ata atg gga 1632
   Lys Leu Ala Thr Glu Leu Tyr His Gln Lys Ser Val Leu Ile Met Gly
   530 535 540
   cga ggc tat cat tat gct act tgt ctt gaa ggg gca ctg aaa atc aaa 1680
   Arg Gly Tyr His Tyr Ala Thr Cys Leu Glu Gly Ala Leu Lys Ile Lys
   545 550 555 560
   gaa att act tat atg cac tct gaa ggc atc ctt gct ggt gaa ttg aaa 1728
   Glu Ile Thr Tyr Met His Ser Glu Gly Ile Leu Ala Gly Glu Leu Lys
   565 570 575
   cat ggc cct ctg gct ttg gtg gat aaa ttg atg cct gtg atc atg atc 1776
   His Gly Pro Leu Ala Leu Val Asp Lys Leu Met Pro Val Ile Met Ile
   580 585 590
   atc atg aga gat cac act tat gcc aag tgt cag aat gct ctt cag caa 1824
   Ile Met Arg Asp His Thr Tyr Ala Lys Cys Gln Asn Ala Leu Gln Gln
   595 600 605
   gtg gtt gct cgg cag ggg cgg cct gtg gta att tgt gat aag gag gat 1872
   Val Val Ala Arg Gln Gly Arg Pro Val Val Ile Cys Asp Lys Glu Asp
   610 615 620
   act gag acc att aag aac aca aaa aga acg atc aag gtg ccc cac tca 1920
   Thr Glu Thr Ile Lys Asn Thr Lys Arg Thr Ile Lys Val Pro His Ser
   625 630 635 640
   gtg gac tgc ttg cag ggc att ctc agc gtg atc cct tta cag ttg ctg 1968
   Val Asp Cys Leu Gln Gly Ile Leu Ser Val Ile Pro Leu Gln Leu Leu
   645 650 655
   gct ttc cac ctt gct gtg ctg aga ggc tat gat gtt gat ttc cca cgg      2016
   Ala Phe His Leu Ala Val Leu Arg Gly Tyr Asp Val Asp Phe Pro Arg
               660                 665                 670
   aat ctt gcc aaa tct gtg act gta gag tga                              2046
   Asn Leu Ala Lys Ser Val Thr Val Glu
           675                 680
<210> 2
   <211> 681
   <212> PRT
   <213> Homo sapiens
<220>
   <221> misc_feature
   <222> (57)..(57)
   <223> Le 'Xaa' en position 57 représente Thr ou Ile.
<400> 2
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Xaa Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
                   85                  90                  95
   Ala Thr His Gly Glu Pro Ser Pro Val Asn Ser His Pro Gln Arg Ser
               100                 105                 110
   Asp Lys Asn Asn Glu Phe Ile Val Ile His Asn Gly Ile Ile Thr Asn
           115                 120                 125
   Tyr Lys Asp Leu Lys Lys Phe Leu Glu Ser Lys Gly Tyr Asp Phe Glu
       130                 135                 140
   Ser Glu Thr Asp Thr Glu Thr Ile Ala Lys Leu Val Lys Tyr Met Tyr
   145                 150                 155                 160
   Asp Asn Arg Glu Ser Gln Asp Thr Ser Phe Thr Thr Leu Val Glu Arg
                   165                 170                 175
   Val Ile Gln Gln Leu Glu Gly Ala Phe Ala Leu Val Phe Lys Ser Val
               180                 185                 190
   His Phe Pro Gly Gln Ala Val Gly Thr Arg Arg Gly Ser Pro Leu Leu
           195                 200                 205
   Ile Gly Val Arg Ser Glu His Lys Leu Ser Thr Asp His Ile Pro Ile
       210                 215                 220
   Leu Tyr Arg Thr Gly Lys Asp Lys Lys Gly Ser Cys Asn Leu Ser Arg
   225                 230                 235                 240
   Val Asp Ser Thr Thr Cys Leu Phe Pro Val Glu Glu Lys Ala Val Glu
                   245                 250                 255
   Tyr Tyr Phe Ala Ser Asp Ala Ser Ala Val Ile Glu His Thr Asn Arg
               260                 265                 270
   Val Ile Phe Leu Glu Asp Asp Asp Val Ala Ala Val Val Asp Gly Arg
           275                 280                 285
   Leu Ser Ile His Arg Ile Lys Arg Thr Ala Gly Asp His Pro Gly Arg
       290                 295                 300
   Ala Val Gln Thr Leu Gln Met Glu Leu Gln Gln Ile Met Lys Gly Asn
   305                 310                 315                 320
   Phe Ser Ser Phe Met Gln Lys Glu Ile Phe Glu Gln Pro Glu Ser Val
                   325                 330                 335
   Val Asn Thr Met Arg Gly Arg Val Asn Phe Asp Asp Tyr Thr Val Asn
               340                 345                 350
   Leu Gly Gly Leu Lys Asp His Ile Lys Glu Ile Gln Arg Cys Arg Arg
           355                 360                 365
   Leu Ile Leu Ile Ala Cys Gly Thr Ser Tyr His Ala Gly Val Ala Thr
       370                 375                 380
   Arg Gln Val Leu Glu Glu Leu Thr Glu Leu Pro Val Met Val Glu Leu
   385                 390                 395                 400
   Ala Ser Asp Phe Leu Asp Arg Asn Thr Pro Val Phe Arg Asp Asp Val
                   405                 410                 415
   Cys Phe Phe Leu Ser Gln Ser Gly Glu Thr Ala Asp Thr Leu Met Gly
               420                 425                 430
   Leu Arg Tyr Cys Lys Glu Arg Gly Ala Leu Thr Val Gly Ile Thr Asn
   435 440 445
   Thr Val Gly Ser Ser Ile Ser Arg Glu Thr Asp Cys Gly Val His Ile
   450 455 460
   Asn Ala Gly Pro Glu Ile Gly Val Ala Ser Thr Lys Ala Tyr Thr Ser
   465 470 475 480
   Gln Phe Val Ser Leu Val Met Phe Ala Leu Met Met Cys Asp Asp Arg
   485 490 495
   Ile Ser Met Gln Glu Arg Arg Lys Glu Ile Met Leu Gly Leu Lys Arg
   500 505 510
   Leu Pro Asp Leu Ile Lys Glu Val Leu Ser Met Asp Asp Glu Ile Gln
   515 520 525
   Lys Leu Ala Thr Glu Leu Tyr His Gln Lys Ser Val Leu Ile Met Gly
   530 535 540
   Arg Gly Tyr His Tyr Ala Thr Cys Leu Glu Gly Ala Leu Lys Ile Lys
   545 550 555 560
   Glu Ile Thr Tyr Met His Ser Glu Gly Ile Leu Ala Gly Glu Leu Lys
   565 570 575
   His Gly Pro Leu Ala Leu Val Asp Lys Leu Met Pro Val Ile Met Ile
   580 585 590
   Ile Met Arg Asp His Thr Tyr Ala Lys Cys Gln Asn Ala Leu Gln Gln
   595 600 605
   Val Val Ala Arg Gln Gly Arg Pro Val Val Ile Cys Asp Lys Glu Asp
   610 615 620
   Thr Glu Thr Ile Lys Asn Thr Lys Arg Thr Ile Lys Val Pro His Ser
   625 630 635 640
   Val Asp Cys Leu Gln Gly Ile Leu Ser Val Ile Pro Leu Gln Leu Leu
   645 650 655
   Ala Phe His Leu Ala Val Leu Arg Gly Tyr Asp Val Asp Phe Pro Arg
   660 665 670
   Asn Leu Ala Lys Ser Val Thr Val Glu
   675 680
<210> 3
   <211> 2049
   <212> ADN
   <213> Homo sapiens
<220>
   <221> CDS
   <222> (1)..(2049)
   <223>
<400> 3
   atg tgc gga atc ttt gcc tac atg aac tac aga gtc ccc cgg acg agg      48
   Met Cys Gly Ile Phe Ala Tyr Met Asn Tyr Arg Val Pro Arg Thr Arg
   1               5                   10                  15
   aag gag atc ttc gaa acc ctc atc aag ggc ctg cag cgg ctg gag tac      96
   Lys Glu Ile Phe Glu Thr Leu Ile Lys Gly Leu Gln Arg Leu Glu Tyr
               20                  25                  30
   aga ggc tac gac tcg gca ggt gtg gcg atc gat ggg aat aat cac gaa     144
   Arg Gly Tyr Asp Ser Ala Gly Val Ala Ile Asp Gly Asn Asn His Glu
           35                  40                  45
   gtc aaa gaa aga cac att cag ctg gtc aag aaa agg ggg aaa gtc aag     192
   Val Lys Glu Arg His Ile Gln Leu Val Lys Lys Arg Gly Lys Val Lys
       50                  55                  60
   gct ctc gat gaa gaa ctt tac aaa caa gac agc atg gac tta aaa gtg     240
   Ala Leu Asp Glu Glu Leu Tyr Lys Gln Asp Ser Met Asp Leu Lys Val
   65                  70                  75                  80
   gag ttt gag aca cac ttc ggc att gcc cac acg cgc tgg gcc acc cac     288
   Glu Phe Glu Thr His Phe Gly Ile Ala His Thr Arg Trp Ala Thr His
                   85                  90                  95
   ggg gtc ccc agt gct gtc aac agc cac cct cag cgc tca gac aaa ggc     336
   Gly Val Pro Ser Ala Val Asn Ser His Pro Gln Arg Ser Asp Lys Gly
               100                 105                 110
   aac gaa ttt gtt gtc atc cac aat ggg atc atc aca aat tac aaa gat     384
   Asn Glu Phe Val Val Ile His Asn Gly Ile Ile Thr Asn Tyr Lys Asp
           115                 120                 125
   ctg agg aaa ttt ctg gaa agc aaa ggc tac gag ttt gag tca gaa aca     432
   Leu Arg Lys Phe Leu Glu Ser Lys Gly Tyr Glu Phe Glu Ser Glu Thr
       130                 135                 140
   gat aca gag acc atc gcc aag ctg att aaa tat gtg ttc gac aac aga     480
   Asp Thr Glu Thr Ile Ala Lys Leu Ile Lys Tyr Val Phe Asp Asn Arg
   145                 150                 155                 160
   gaa act gag gac att acg ttt tca acg ttg gtc gag aga gtc att cag     528
   Glu Thr Glu Asp Ile Thr Phe Ser Thr Leu Val Glu Arg Val Ile Gln
                   165                 170                 175
   cag ttg gaa ggt gca ttc gcg ctg gtt ttc aag agt gtc cac tac cca     576
   Gln Leu Glu Gly Ala Phe Ala Leu Val Phe Lys Ser Val His Tyr Pro
               180                 185                 190
   gga gaa gcc gtt gcc aca cgg aga ggc agc ccc ctg ctc atc gga gtc     624
   Gly Glu Ala Val Ala Thr Arg Arg Gly Ser Pro Leu Leu Ile Gly Val
           195                 200                 205
   cgg agc aaa tac aag ctc tcc aca gaa cag atc cct atc tta tac agg     672
   Arg Ser Lys Tyr Lys Leu Ser Thr Glu Gln Ile Pro Ile Leu Tyr Arg
       210                 215                 220
   acg tgc act ctg gag aat gtg aag aat atc tgt aag aca cgg atg aag 720
   Thr Cys Thr Leu Glu Asn Val Lys Asn Ile Cys Lys Thr Arg Met Lys
   225 230 235 240
   agg ctg gac agc tcc gcc tgc ctg cat gct gtg ggc gac aag gcc gtg 768
   Arg Leu Asp Ser Ser Ala Cys Leu His Ala Val Gly Asp Lys Ala Val
   245 250 255
   gaa ttc ttc ttt gct tct gat gca agc gct atc ata gag cac acc aac 816
   Glu Phe Phe Phe Ala Ser Asp Ala Ser Ala Ile Ile Glu His Thr Asn
   260 265 270
   cgg gtc atc ttc ctg gag gac gat gac atc gcc gca gtg gct gat ggg 864
   Arg Val Ile Phe Leu Glu Asp Asp Asp Ile Ala Ala Val Ala Asp Gly
   275 280 285
   aaa ctc tcc att cac cgg gtc aag cgc tcg gcc agt gat gac cca tct 912
   Lys Leu Ser Ile His Arg Val Lys Arg Ser Ala Ser Asp Asp Pro Ser
   290 295 300
   cga gcc atc cag acc ttg cag atg gaa ctg cag caa atc atg aaa ggt 960
   Arg Ala Ile Gln Thr Leu Gln Met Glu Leu Gln Gln Ile Met Lys Gly
   305 310 315 320
   aac ttc agt gcg ttt atg cag aag gag atc ttc gaa cag cca gaa tca 1008
   Asn Phe Ser Ala Phe Met Gln Lys Glu Ile Phe Glu Gln Pro Glu Ser
   325 330 335
   gtt ttc aat act atg aga ggt cgg gtg aat ttt gaa acc aac aca gtg 1056
   Val Phe Asn Thr Met Arg Gly Arg Val Asn Phe Glu Thr Asn Thr Val
   340 345 350
   ctc ctg ggt ggc ttg aag gac cac ttg aag gag att cga cga tgc cga 1104
   Leu Leu Gly Gly Leu Lys Asp His Leu Lys Glu Ile Arg Arg Cys Arg
   355 360 365
   cgg ctc atc gtg att ggc tgt gga acc agc tac cac gct gcc gtg gct 1152
   Arg Leu Ile Val Ile Gly Cys Gly Thr Ser Tyr His Ala Ala Val Ala
   370 375 380
   acg cgg caa gtt ttg gag gaa ctg act gag ctt cct gtg atg gtt gaa 1200
   Thr Arg Gln Val Leu Glu Glu Leu Thr Glu Leu Pro Val Met Val Glu
   385 390 395 400
   ctt gct agt gat ttt ctg gac agg aac aca cct gtg ttc agg gat gac 1248
   Leu Ala Ser Asp Phe Leu Asp Arg Asn Thr Pro Val Phe Arg Asp Asp
   405 410 415
   gtt tgc ttt ttc atc agc cag tca ggc gag acc gcg gac acc ctc ctg 1296
   Val Cys Phe Phe Ile Ser Gln Ser Gly Glu Thr Ala Asp Thr Leu Leu
   420 425 430
   gcg ctg cgc tac tgt aag gac cgc ggc gct ctc acc gtg ggc gtc acc 1344
   Ala Leu Arg Tyr Cys Lys Asp Arg Gly Ala Leu Thr Val Gly Val Thr
   435 440 445
   aac acc gtg ggc agc tcc atc tct cgc gag acc gac tgc ggc gtc cac 1392
   Asn Thr Val Gly Ser Ser Ile Ser Arg Glu Thr Asp Cys Gly Val His
   450 455 460
   atc aac gca ggg ccg gag gtc ggc gtg gcc agc acc aag gct tat acc 1440
   Ile Asn Ala Gly Pro Glu Val Gly Val Ala Ser Thr Lys Ala Tyr Thr
   465 470 475 480
   agt cag ttc atc tct ctg gtg atg ttt ggt ttg atg atg tct gaa gac 1488
   Ser Gln Phe Ile Ser Leu Val Met Phe Gly Leu Met Met Ser Glu Asp
                   485                 490                 495
   cga att tca cta caa aac agg agg caa gag atc atc cgt ggc ttg aga      1536
   Arg Ile Ser Leu Gln Asn Arg Arg Gln Glu Ile Ile Arg Gly Leu Arg
               500                 505                 510
   tct tta cct gag ctg atc aag gaa gtg ctg tct ctg gag gag aag atc      1584
   Ser Leu Pro Glu Leu Ile Lys Glu Val Leu Ser Leu Glu Glu Lys Ile
           515                 520                 525
   cac gac ttg gcc ctg gag ctc tac acg cag aga tcg ctg ctg gtg atg      1632
   His Asp Leu Ala Leu Glu Leu Tyr Thr Gln Arg Ser Leu Leu Val Met
       530                 535                 540
   ggg cgg ggc tac aac tat gcc acc tgc ctg gaa gga gcc ctg aaa att      1680
   Gly Arg Gly Tyr Asn Tyr Ala Thr Cys Leu Glu Gly Ala Leu Lys Ile
   545                 550                 555                 560
   aaa gag ata acc tac atg cac tca gaa ggc atc ctg gct ggg gag ctg      1728
   Lys Glu Ile Thr Tyr Met His Ser Glu Gly Ile Leu Ala Gly Glu Leu
                   565                 570                 575
   aag cac ggg ccc ctg gca ctg att gac aag cag atg ccc gtc atc atg      1776
   Lys His Gly Pro Leu Ala Leu Ile Asp Lys Gln Met Pro Val Ile Met
               580                 585                 590
   gtc att atg aag gat cct tgc ttc gcc aaa tgc cag aac gcc ctg cag      1824
   Val Ile Met Lys Asp Pro Cys Phe Ala Lys Cys Gln Asn Ala Leu Gln
           595                 600                 605
   caa gtc acg gcc cgc cag ggt cgc ccc att ata ctg tgc tcc aag gac      1872
   Gln Val Thr Ala Arg Gln Gly Arg Pro Ile Ile Leu Cys Ser Lys Asp
       610                 615                 620
   gat act gaa agt tcc aag ttt gcg tat aag aca atc gag ctg ccc cac      1920
   Asp Thr Glu Ser Ser Lys Phe Ala Tyr Lys Thr Ile Glu Leu Pro His
   625                 630                 635                 640
   act gtg gac tgc ctc cag ggc atc ctg agc gtg att ccg ctg cag ctg      1968
   Thr Val Asp Cys Leu Gln Gly Ile Leu Ser Val Ile Pro Leu Gln Leu
                   645                 650                 655
   ctg tcc ttc cac ctg gct gtt ctc cga gga tat gac gtt gac ttc ccc      2016
   Leu Ser Phe His Leu Ala Val Leu Arg Gly Tyr Asp Val Asp Phe Pro
               660                 665                 670
   aga aat ctg gcc aag tct gta act gtg gaa tga                          2049
   Arg Asn Leu Ala Lys Ser Val Thr Val Glu
           675                 680
<210> 4
   <211> 682
   <212> PRT
   <213> Homo sapiens
<400> 4
   Met Cys Gly Ile Phe Ala Tyr Met Asn Tyr Arg Val Pro Arg Thr Arg
   1 5 10 15
   Lys Glu Ile Phe Glu Thr Leu Ile Lys Gly Leu Gln Arg Leu Glu Tyr
   20 25 30
   Arg Gly Tyr Asp Ser Ala Gly Val Ala Ile Asp Gly Asn Asn His Glu
           35                  40                  45
   Val Lys Glu Arg His Ile Gln Leu Val Lys Lys Arg Gly Lys Val Lys
       50                  55                  60
   Ala Leu Asp Glu Glu Leu Tyr Lys Gln Asp Ser Met Asp Leu Lys Val
   65                  70                  75                  80
   Glu Phe Glu Thr His Phe Gly Ile Ala His Thr Arg Trp Ala Thr His
                   85                  90                  95
   Gly Val Pro Ser Ala Val Asn Ser His Pro Gln Arg Ser Asp Lys Gly
               100                 105                 110
   Asn Glu Phe Val Val Ile His Asn Gly Ile Ile Thr Asn Tyr Lys Asp
           115                 120                 125
   Leu Arg Lys Phe Leu Glu Ser Lys Gly Tyr Glu Phe Glu Ser Glu Thr
       130                 135                 140
   Asp Thr Glu Thr Ile Ala Lys Leu Ile Lys Tyr Val Phe Asp Asn Arg
   145                 150                 155                 160
   Glu Thr Glu Asp Ile Thr Phe Ser Thr Leu Val Glu Arg Val Ile Gln
                   165                 170                 175
   Gln Leu Glu Gly Ala Phe Ala Leu Val Phe Lys Ser Val His Tyr Pro
               180                 185                 190
   Gly Glu Ala Val Ala Thr Arg Arg Gly Ser Pro Leu Leu Ile Gly Val
           195                 200                 205
   Arg Ser Lys Tyr Lys Leu Ser Thr Glu Gln Ile Pro Ile Leu Tyr Arg
       210                 215                 220
   Thr Cys Thr Leu Glu Asn Val Lys Asn Ile Cys Lys Thr Arg Met Lys
   225                 230                 235                 240
   Arg Leu Asp Ser Ser Ala Cys Leu His Ala Val Gly Asp Lys Ala Val
                   245                 250                 255
   Glu Phe Phe Phe Ala Ser Asp Ala Ser Ala Ile Ile Glu His Thr Asn
               260                 265                 270
   Arg Val Ile Phe Leu Glu Asp Asp Asp Ile Ala Ala Val Ala Asp Gly
           275                 280                 285
   Lys Leu Ser Ile His Arg Val Lys Arg Ser Ala Ser Asp Asp Pro Ser
       290                 295                 300
   Arg Ala Ile Gln Thr Leu Gln Met Glu Leu Gln Gln Ile Met Lys Gly
   305                 310                 315                 320
   Asn Phe Ser Ala Phe Met Gln Lys Glu Ile Phe Glu Gln Pro Glu Ser
                   325                 330                 335
   Val Phe Asn Thr Met Arg Gly Arg Val Asn Phe Glu Thr Asn Thr Val
               340                 345                 350
   Leu Leu Gly Gly Leu Lys Asp His Leu Lys Glu Ile Arg Arg Cys Arg
           355                 360                 365
   Arg Leu Ile Val Ile Gly Cys Gly Thr Ser Tyr His Ala Ala Val Ala
       370                 375                 380
   Thr Arg Gln Val Leu Glu Glu Leu Thr Glu Leu Pro Val Met Val Glu
   385                 390                 395                 400
   Leu Ala Ser Asp Phe Leu Asp Arg Asn Thr Pro Val Phe Arg Asp Asp
                   405                 410                 415
   Val Cys Phe Phe Ile Ser Gln Ser Gly Glu Thr Ala Asp Thr Leu Leu
               420                 425                 430
   Ala Leu Arg Tyr Cys Lys Asp Arg Gly Ala Leu Thr Val Gly Val Thr
           435                 440                 445
   Asn Thr Val Gly Ser Ser Ile Ser Arg Glu Thr Asp Cys Gly Val His
       450                 455                 460
   Ile Asn Ala Gly Pro Glu Val Gly Val Ala Ser Thr Lys Ala Tyr Thr
   465                 470                 475                 480
   Ser Gln Phe Ile Ser Leu Val Met Phe Gly Leu Met Met Ser Glu Asp
                   485                 490                 495
   Arg Ile Ser Leu Gln Asn Arg Arg Gln Glu Ile Ile Arg Gly Leu Arg
               500                 505                 510
   Ser Leu Pro Glu Leu Ile Lys Glu Val Leu Ser Leu Glu Glu Lys Ile
           515                 520                 525
   His Asp Leu Ala Leu Glu Leu Tyr Thr Gln Arg Ser Leu Leu Val Met
       530                 535                 540
   Gly Arg Gly Tyr Asn Tyr Ala Thr Cys Leu Glu Gly Ala Leu Lys Ile
   545 550 555 560
   Lys Glu Ile Thr Tyr Met His Ser Glu Gly Ile Leu Ala Gly Glu Leu
   565 570 575
   Lys His Gly Pro Leu Ala Leu Ile Asp Lys Gln Met Pro Val Ile Met
   580 585 590
   Val Ile Met Lys Asp Pro Cys Phe Ala Lys Cys Gln Asn Ala Leu Gln
   595 600 605
   Gln Val Thr Ala Arg Gln Gly Arg Pro Ile Ile Leu Cys Ser Lys Asp
   610 615 620
   Asp Thr Glu Ser Ser Lys Phe Ala Tyr Lys Thr Ile Glu Leu Pro His
   625 630 635 640
   Thr Val Asp Cys Leu Gln Gly Ile Leu Ser Val Ile Pro Leu Gln Leu
   645 650 655
   Leu Ser Phe His Leu Ala Val Leu Arg Gly Tyr Asp Val Asp Phe Pro
   660 665 670
   Arg Asn Leu Ala Lys Ser Val Thr Val Glu
   675 680
<210> 5
   <211> 2100
   <212> ADN
   <213> Homo sapiens
<220>
   <221> CDS
   <222> (1) .. (2100)
   <223>
<220>
   <221> misc_feature
   <222> (170)..(170)
   <223> t ou c
<400> 5
   atg tgt ggt ata ttt gct tac tta aac tac cat gtt cct cga acg aga      48
   Met Cys Gly Ile Phe Ala Tyr Leu Asn Tyr His Val Pro Arg Thr Arg
   1               5                   10                  15
   cga gaa atc ctg gag acc cta atc aaa ggc ctt cag aga ctg gag tac      96
   Arg Glu Ile Leu Glu Thr Leu Ile Lys Gly Leu Gln Arg Leu Glu Tyr
               20                  25                  30
   aga gga tat gat tct gct ggt gtg gga ttt gat gga ggc aat gat aaa     144
   Arg Gly Tyr Asp Ser Ala Gly Val Gly Phe Asp Gly Gly Asn Asp Lys
           35                  40                  45
   gat tgg gaa gcc aat gcc tgc aaa anc cag ctt att aag aag aaa gga      192
   Asp Trp Glu Ala Asn Ala Cys Lys Xaa Gln Leu Ile Lys Lys Lys Gly
       50                  55                  60
   aaa gtt aag gca ctg gat gaa gaa gtt cac aag caa caa gat atg gat      240
   Lys Val Lys Ala Leu Asp Glu Glu Val His Lys Gln Gln Asp Met Asp
   65                  70                  75                  80
   ttg gat ata gaa ttt gat gta cac ctt gga ata gct cat acc cgt tgg      288
   Leu Asp Ile Glu Phe Asp Val His Leu Gly Ile Ala His Thr Arg Trp
                   85                  90                  95
   gca aca cat gga gaa ccc agt cct gtc aat agc cac ccc cag cgc tct      336
   Ala Thr His Gly Glu Pro Ser Pro Val Asn Ser His Pro Gln Arg Ser
               100                 105                 110
   gat aaa aat aat gaa ttt atc gtt att cac aat gga atc atc acc aac      384
   Asp Lys Asn Asn Glu Phe Ile Val Ile His Asn Gly Ile Ile Thr Asn
           115                 120                 125
   tac aaa gac ttg aaa aag ttt ttg gaa agc aaa ggc tat gac ttc gaa      432
   Tyr Lys Asp Leu Lys Lys Phe Leu Glu Ser Lys Gly Tyr Asp Phe Glu
       130                 135                 140
   tct gaa aca gac aca gag aca att gcc aag ctc gtt aag tat atg tat      480
   Ser Glu Thr Asp Thr Glu Thr Ile Ala Lys Leu Val Lys Tyr Met Tyr
   145                 150                 155                 160
   gac aat cgg gaa agt caa gat acc agc ttt act acc ttg gtg gag aga      528
   Asp Asn Arg Glu Ser Gln Asp Thr Ser Phe Thr Thr Leu Val Glu Arg
                   165                 170                 175
   gtt atc caa caa ttg gaa ggt gct ttt gca ctt gtg ttt aaa agt gtt      576
   Val Ile Gln Gln Leu Glu Gly Ala Phe Ala Leu Val Phe Lys Ser Val
               180                 185                 190
   cat ttt ccc ggg caa gca gtt ggc aca agg cga ggt agc cct ctg ttg      624
   His Phe Pro Gly Gln Ala Val Gly Thr Arg Arg Gly Ser Pro Leu Leu
           195                 200                 205
   att ggt gta cgg agt gaa cat aaa ctt tct act gat cac att cct ata      672
   Ile Gly Val Arg Ser Glu His Lys Leu Ser Thr Asp His Ile Pro Ile
       210                 215                 220
   ctc tac aga aca gct agg act cag att gga tca aaa ttc aca cgg tgg      720
   Leu Tyr Arg Thr Ala Arg Thr Gln Ile Gly Ser Lys Phe Thr Arg Trp
   225                 230                 235                 240
   gga tca cag gga gaa aga ggc aaa gac aag aaa gga agc tgc aat ctc      768
   Gly Ser Gln Gly Glu Arg Gly Lys Asp Lys Lys Gly Ser Cys Asn Leu
                   245                 250                 255
   tct cgt gtg gac agc aca acc tgc ctt ttc ccg gtg gaa gaa aaa gca      816
   Ser Arg Val Asp Ser Thr Thr Cys Leu Phe Pro Val Glu Glu Lys Ala
               260                 265                 270
   gtg gag tat tac ttt gct tct gat gca agt gct gtc ata gaa cac acc      864
   Val Glu Tyr Tyr Phe Ala Ser Asp Ala Ser Ala Val Ile Glu His Thr
           275                 280                 285
   aat cgc gtc atc ttt ctg gaa gat gat gat gtt gca gca gta gtg gat      912
   Asn Arg Val Ile Phe Leu Glu Asp Asp Asp Val Ala Ala Val Val Asp
       290                 295                 300
   gga cgt ctt tct atc cat cga att aaa cga act gca gga gat cac ccc      960
   Gly Arg Leu Ser Ile His Arg Ile Lys Arg Thr Ala Gly Asp His Pro
   305                 310                 315                 320
   gga cga gct gtg caa aca ctc cag atg gaa ctc cag cag atc atg aag     1008
   Gly Arg Ala Val Gln Thr Leu Gln Met Glu Leu Gln Gln Ile Met Lys
                   325                 330                 335
   ggc aac ttc agt tca ttt atg cag aag gaa ata ttt gag cag cca gag     1056
   Gly Asn Phe Ser Ser Phe Met Gln Lys Glu Ile Phe Glu Gln Pro Glu
               340                 345                 350
   tct gtc gtg aac aca atg aga gga aga gtc aac ttt gat gac tat act     1104
   Ser Val Val Asn Thr Met Arg Gly Arg Val Asn Phe Asp Asp Tyr Thr
           355                 360                 365
   gtg aat ttg ggt ggt ttg aag gat cac ata aag gag atc cag aga tgc     1152
   Val Asn Leu Gly Gly Leu Lys Asp His Ile Lys Glu Ile Gln Arg Cys
       370                 375                 380
   cgg cgt ttg att ctt att gct tgt gga aca agt tac cat gct ggt gta     1200
   Arg Arg Leu Ile Leu Ile Ala Cys Gly Thr Ser Tyr His Ala Gly Val
   385                 390                 395                 400
   gca aca cgt caa gtt ctt gag gag ctg act gag ttg cct gtg atg gtg     1248
   Ala Thr Arg Gln Val Leu Glu Glu Leu Thr Glu Leu Pro Val Met Val
                   405                 410                 415
   gaa cta gca agt gac ttc ctg gac aga aac aca cca gtc ttt cga gat     1296
   Glu Leu Ala Ser Asp Phe Leu Asp Arg Asn Thr Pro Val Phe Arg Asp
               420                 425                 430
   gat gtt tgc ttt ttc ctt agt caa tca ggt gag aca gca gat act ttg     1344
   Asp Val Cys Phe Phe Leu Ser Gln Ser Gly Glu Thr Ala Asp Thr Leu
           435                 440                 445
   atg ggt ctt cgt tac tgt aag gag aga gga gct tta act gtg ggg atc     1392
   Met Gly Leu Arg Tyr Cys Lys Glu Arg Gly Ala Leu Thr Val Gly Ile
       450                 455                 460
   aca aac aca gtt ggc agt tcc ata tca cgg gag aca gat tgt gga gtt     1440
   Thr Asn Thr Val Gly Ser Ser Ile Ser Arg Glu Thr Asp Cys Gly Val
   465                 470                 475                 480
   cat att aat gct ggt cct gag att ggt gtg gcc agt aca aag gct tat     1488
   His Ile Asn Ala Gly Pro Glu Ile Gly Val Ala Ser Thr Lys Ala Tyr
                   485                 490                 495
   acc agc cag ttt gta tcc ctt gtg atg ttt gcc ctt atg atg tgt gat     1536
   Thr Ser Gln Phe Val Ser Leu Val Met Phe Ala Leu Met Met Cys Asp
               500                 505                 510
   gat cgg atc tcc atg caa gaa aga cgc aaa gag atc atg ctt gga ttg     1584
   Asp Arg Ile Ser Met Gln Glu Arg Arg Lys Glu Ile Met Leu Gly Leu
           515                 520                 525
   aaa cgg ctg cct gat ttg att aag gaa gta ctg agc atg gat gac gaa     1632
   Lys Arg Leu Pro Asp Leu Ile Lys Glu Val Leu Ser Met Asp Asp Glu
       530                 535                 540
   att cag aaa cta gca aca gaa ctt tat cat cag aag tca gtt ctg ata     1680
   Ile Gln Lys Leu Ala Thr Glu Leu Tyr His Gln Lys Ser Val Leu Ile
   545                 550                 555                 560
   atg gga cga ggc tat cat tat gct act tgt ctt gaa ggg gca ctg aaa     1728
   Met Gly Arg Gly Tyr His Tyr Ala Thr Cys Leu Glu Gly Ala Leu Lys
                       565                 570                 575
   atc aaa gaa att act tat atg cac tct gaa ggc atc ctt gct ggt gaa        1776
   Ile Lys Glu Ile Thr Tyr Met His Ser Glu Gly Ile Leu Ala Gly Glu
               580                 585                 590
   ttg aaa cat ggc cct ctg gct ttg gtg gat aaa ttg atg cct gtg atc        1824
   Leu Lys His Gly Pro Leu Ala Leu Val Asp Lys Leu Met Pro Val Ile
           595                 600                 605
   atg atc atc atg aga gat cac act tat gcc aag tgt cag aat gct ctt        1872
   Met Ile Ile Met Arg Asp His Thr Tyr Ala Lys Cys Gln Asn Ala Leu
       610                 615                 620
   cag caa gtg gtt gct cgg cag ggg cgg cct gtg gta att tgt gat aag        1920
   Gln Gln Val Val Ala Arg Gln Gly Arg Pro Val Val Ile Cys Asp Lys
   625                 630                 635                 640
   gag gat act gag acc att aag aac aca aaa aga acg atc aag gtg ccc        1968
   Glu Asp Thr Glu Thr Ile Lys Asn Thr Lys Arg Thr Ile Lys Val Pro
                   645                 650                 655
   cac tca gtg gac tgc ttg cag ggc att ctc agc gtg atc cct tta cag        2016
   His Ser Val Asp Cys Leu Gln Gly Ile Leu Ser Val Ile Pro Leu Gln
               660                 665                 670
   ttg ctg gct ttc cac ctt gct gtg ctg aga ggc tat gat gtt gat ttc        2064
   Leu Leu Ala Phe His Leu Ala Val Leu Arg Gly Tyr Asp Val Asp Phe
           675                 680                 685
   cca cgg aat ctt gcc aaa tct gtg act gta gag tga                        2100
   Pro Arg Asn Leu Ala Lys Ser Val Thr Val Glu
       690                 695
<210> 6
   <211> 699
   <212> PRT
   <213> Homo sapiens
<220>
   <221> misc_feature
   <222> (57).. (57)
   <223> Le 'Xaa' en position 57 représente Thr ou Ile.
<400> 6
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Xaa Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
   85 90 95
   Ala Thr His Gly Glu Pro Ser Pro Val Asn Ser His Pro Gln Arg Ser
   100 105 110
   Asp Lys Asn Asn Glu Phe Ile Val Ile His Asn Gly Ile Ile Thr Asn
   115 120 125
   Tyr Lys Asp Leu Lys Lys Phe Leu Glu Ser Lys Gly Tyr Asp Phe Glu
   130 135 140
   Ser Glu Thr Asp Thr Glu Thr Ile Ala Lys Leu Val Lys Tyr Met Tyr
   145 150 155 160
   Asp Asn Arg Glu Ser Gln Asp Thr Ser Phe Thr Thr Leu Val Glu Arg
   165 170 175
   Val Ile Gln Gln Leu Glu Gly Ala Phe Ala Leu Val Phe Lys Ser Val
   180 185 190
   His Phe Pro Gly Gln Ala Val Gly Thr Arg Arg Gly Ser Pro Leu Leu
   195 200 205
   Ile Gly Val Arg Ser Glu His Lys Leu Ser Thr Asp His Ile Pro Ile
   210 215 220
   Leu Tyr Arg Thr Ala Arg Thr Gln Ile Gly Ser Lys Phe Thr Arg Trp
   225 230 235 240
   Gly Ser Gln Gly Glu Arg Gly Lys Asp Lys Lys Gly Ser Cys Asn Leu
   245 250 255
   Ser Arg Val Asp Ser Thr Thr Cys Leu Phe Pro Val Glu Glu Lys Ala
   260 265 270
   Val Glu Tyr Tyr Phe Ala Ser Asp Ala Ser Ala Val Ile Glu His Thr
   275 280 285
   Asn Arg Val Ile Phe Leu Glu Asp Asp Asp Val Ala Ala Val Val Asp
   290 295 300
   Gly Arg Leu Ser Ile His Arg Ile Lys Arg Thr Ala Gly Asp His Pro
   305 310 315 320
   Gly Arg Ala Val Gln Thr Leu Gln Met Glu Leu Gln Gln Ile Met Lys
   325 330 335
   Gly Asn Phe Ser Ser Phe Met Gln Lys Glu Ile Phe Glu Gln Pro Glu
   340 345 350
   Ser Val Val Asn Thr Met Arg Gly Arg Val Asn Phe Asp Asp Tyr Thr
   355 360 365
   Val Asn Leu Gly Gly Leu Lys Asp His Ile Lys Glu Ile Gln Arg Cys
   370 375 380
   Arg Arg Leu Ile Leu Ile Ala Cys Gly Thr Ser Tyr His Ala Gly Val
   385 390 395 400
   Ala Thr Arg Gln Val Leu Glu Glu Leu Thr Glu Leu Pro Val Met Val
   405 410 415
   Glu Leu Ala Ser Asp Phe Leu Asp Arg Asn Thr Pro Val Phe Arg Asp
   420 425 430
   Asp Val Cys Phe Phe Leu Ser Gln Ser Gly Glu Thr Ala Asp Thr Leu
   435 440 445
   Met Gly Leu Arg Tyr Cys Lys Glu Arg Gly Ala Leu Thr Val Gly Ile
   450 455 460
   Thr Asn Thr Val Gly Ser Ser Ile Ser Arg Glu Thr Asp Cys Gly Val
   465 470 475 480
   His Ile Asn Ala Gly Pro Glu Ile Gly Val Ala Ser Thr Lys Ala Tyr
   485 490 495
   Thr Ser Gln Phe Val Ser Leu Val Met Phe Ala Leu Met Met Cys Asp
   500 505 510
   Asp Arg Ile Ser Met Gln Glu Arg Arg Lys Glu Ile Met Leu Gly Leu
   515 520 525
   Lys Arg Leu Pro Asp Leu Ile Lys Glu Val Leu Ser Met Asp Asp Glu
   530 535 540
   Ile Gln Lys Leu Ala Thr Glu Leu Tyr His Gln Lys Ser Val Leu Ile
   545 550 555 560
   Met Gly Arg Gly Tyr His Tyr Ala Thr Cys Leu Glu Gly Ala Leu Lys
   565 570 575
   Ile Lys Glu Ile Thr Tyr Met His Ser Glu Gly Ile Leu Ala Gly Glu
   580 585 590
   Leu Lys His Gly Pro Leu Ala Leu Val Asp Lys Leu Met Pro Val Ile
   595 600 605
   Met Ile Ile Met Arg Asp His Thr Tyr Ala Lys Cys Gln Asn Ala Leu
   610 615 620
   Gln Gln Val Val Ala Arg Gln Gly Arg Pro Val Val Ile Cys Asp Lys
   625 630 635 640
   Glu Asp Thr Glu Thr Ile Lys Asn Thr Lys Arg Thr Ile Lys Val Pro
   645 650 655
   His Ser Val Asp Cys Leu Gln Gly Ile Leu Ser Val Ile Pro Leu Gln
   660 665 670
   Leu Leu Ala Phe His Leu Ala Val Leu Arg Gly Tyr Asp Val Asp Phe
   675 680 685
   Pro Arg Asn Leu Ala Lys Ser Val Thr Val Glu
   690 695
<210> 7
   <211> 2064
   <212> ADN
   <213> Séquence artificielle
<220>
   <223> GFAT1 modifiée par une étiquette de purification interne
<220>
   <221> CDS
   <222> (1) .. (2064)
   <223>
<220>
   <221> misc_feature
   <222> (170)..(170)
   <223> t ou c
<400> 7
   atg tgt ggt ata ttt gct tac tta aac tac cat gtt cct cga acg aga        48
   Met Cys Gly Ile Phe Ala Tyr Leu Asn Tyr His Val Pro Arg Thr Arg
   1               5                   10                  15
   cga gaa atc ctg gag acc cta atc aaa ggc ctt cag aga ctg gag tac        96
   Arg Glu Ile Leu Glu Thr Leu Ile Lys Gly Leu Gln Arg Leu Glu Tyr
               20                  25                  30
   aga gga tat gat tct gct ggt gtg gga ttt gat gga ggc aat gat aaa       144
   Arg Gly Tyr Asp Ser Ala Gly Val Gly Phe Asp Gly Gly Asn Asp Lys
           35                  40                  45
   gat tgg gaa gcc aat gcc tgc aaa anc cag ctt att aag aag aaa gga       192
   Asp Trp Glu Ala Asn Ala Cys Lys Xaa Gln Leu Ile Lys Lys Lys Gly
       50                  55                  60
   aaa gtt aag gca ctg gat gaa gaa gtt cac aag caa caa gat atg gat       240
   Lys Val Lys Ala Leu Asp Glu Glu Val His Lys Gln Gln Asp Met Asp
   65                  70                  75                  80
   ttg gat ata gaa ttt gat gta cac ctt gga ata gct cat acc cgt tgg     288
   Leu Asp Ile Glu Phe Asp Val His Leu Gly Ile Ala His Thr Arg Trp
                   85                  90                  95
   gca aca cat gga gaa ccc agt cct gtc aat agc cac ccc cag cgc tct     336
   Ala Thr His Gly Glu Pro Ser Pro Val Asn Ser His Pro Gln Arg Ser
               100                 105                 110
   gat aaa aat aat gaa ttt atc gtt att cac aat gga atc atc acc aac     384
   Asp Lys Asn Asn Glu Phe Ile Val Ile His Asn Gly Ile Ile Thr Asn
           115                 120                 125
   tac aaa gac ttg aaa aag ttt ttg gaa agc aaa ggc tat gac ttc gaa     432
   Tyr Lys Asp Leu Lys Lys Phe Leu Glu Ser Lys Gly Tyr Asp Phe Glu
       130                 135                 140
   tct gaa aca gac aca gag aca att gcc aag ctc gtt aag tat atg tat     480
   Ser Glu Thr Asp Thr Glu Thr Ile Ala Lys Leu Val Lys Tyr Met Tyr
   145                 150                 155                 160
   gac aat cgg gaa agt caa gat acc agc ttt act acc ttg gtg gag aga     528
   Asp Asn Arg Glu Ser Gln Asp Thr Ser Phe Thr Thr Leu Val Glu Arg
                   165                 170                 175
   gtt atc caa caa ttg gaa ggt gct ttt gca ctt gtg ttt aaa agt gtt     576
   Val Ile Gln Gln Leu Glu Gly Ala Phe Ala Leu Val Phe Lys Ser Val
               180                 185                 190
   cat ttt ccc ggg caa gca gtt ggc aca agg cga ggt agc cct ctg ttg     624
   His Phe Pro Gly Gln Ala Val Gly Thr Arg Arg Gly Ser Pro Leu Leu
           195                 200                 205
   att ggt gta cgg agt gaa cat aaa ctt tct act gat cac att cct ata     672
   Ile Gly Val Arg Ser Glu His Lys Leu Ser Thr Asp His Ile Pro Ile
       210                 215                 220
   ctc tac aga aca ggc aaa gac aag aaa gga agc tgc aat ctc tct cgt     720
   Leu Tyr Arg Thr Gly Lys Asp Lys Lys Gly Ser Cys Asn Leu Ser Arg
   225                 230                 235                 240
   gtg gac agc aca acc tgc ctt ttc ccg gtg gaa gaa aaa gca gtg gag     768
   Val Asp Ser Thr Thr Cys Leu Phe Pro Val Glu Glu Lys Ala Val Glu
                   245                 250                 255
   tat tac ttt gct tct gat gca agt gct gtc ata gaa cac acc aat cgc     816
   Tyr Tyr Phe Ala Ser Asp Ala Ser Ala Val Ile Glu His Thr Asn Arg
               260                 265                 270
   gtc atc ttt ctg gaa gat gat gat gtt gca gca gta gtg gat gga cgt     864
   Val Ile Phe Leu Glu Asp Asp Asp Val Ala Ala Val Val Asp Gly Arg
           275                 280                 285
   ctt tct atc cat cga att aaa cga act gca gga cat cac cat cac cat     912
   Leu Ser Ile His Arg Ile Lys Arg Thr Ala Gly His His His His His
       290                 295                 300
   cac gat cac ccc gga cga gct gtg caa aca ctc cag atg gaa ctc cag     960
   His Asp His Pro Gly Arg Ala Val Gln Thr Leu Gln Met Glu Leu Gln
   305                 310                 315                 320
   cag atc atg aag ggc aac ttc agt tca ttt atg cag aag gaa ata ttt    1008
   Gln Ile Met Lys Gly Asn Phe Ser Ser Phe Met Gln Lys Glu Ile Phe
                   325                 330                 335
   gag cag cca gag tct gtc gtg aac aca atg aga gga aga gtc aac ttt      1056
   Glu Gln Pro Glu Ser Val Val Asn Thr Met Arg Gly Arg Val Asn Phe
               340                 345                 350
   gat gac tat act gtg aat ttg ggt ggt ttg aag gat cac ata aag gag      1104
   Asp Asp Tyr Thr Val Asn Leu Gly Gly Leu Lys Asp His Ile Lys Glu
           355                 360                 365
   atc cag aga tgc cgg cgt ttg att ctt att gct tgt gga aca agt tac      1152
   Ile Gln Arg Cys Arg Arg Leu Ile Leu Ile Ala Cys Gly Thr Ser Tyr
       370                 375                 380
   cat gct ggt gta gca aca cgt caa gtt ctt gag gag ctg act gag ttg      1200
   His Ala Gly Val Ala Thr Arg Gln Val Leu Glu Glu Leu Thr Glu Leu
   385                 390                 395                 400
   cct gtg atg gtg gaa cta gca agt gac ttc ctg gac aga aac aca cca      1248
   Pro Val Met Val Glu Leu Ala Ser Asp Phe Leu Asp Arg Asn Thr Pro
                   405                 410                 415
   gtc ttt cga gat gat gtt tgc ttt ttc ctt agt caa tca ggt gag aca      1296
   Val Phe Arg Asp Asp Val Cys Phe Phe Leu Ser Gln Ser Gly Glu Thr
               420                 425                 430
   gca gat act ttg atg ggt ctt cgt tac tgt aag gag aga gga gct tta      1344
   Ala Asp Thr Leu Met Gly Leu Arg Tyr Cys Lys Glu Arg Gly Ala Leu
           435                 440                 445
   act gtg ggg atc aca aac aca gtt ggc agt tcc ata tca cgg gag aca      1392
   Thr Val Gly Ile Thr Asn Thr Val Gly Ser Ser Ile Ser Arg Glu Thr
       450                 455                 460
   gat tgt gga gtt cat att aat gct ggt cct gag att ggt gtg gcc agt      1440
   Asp Cys Gly Val His Ile Asn Ala Gly Pro Glu Ile Gly Val Ala Ser
   465                 470                 475                 480
   aca aag gct tat acc agc cag ttt gta tcc ctt gtg atg ttt gcc ctt      1488
   Thr Lys Ala Tyr Thr Ser Gln Phe Val Ser Leu Val Met Phe Ala Leu
                   485                 490                 495
   atg atg tgt gat gat cgg atc tcc atg caa gaa aga cgc aaa gag atc      1536
   Met Met Cys Asp Asp Arg Ile Ser Met Gln Glu Arg Arg Lys Glu Ile
               500                 505                 510
   atg ctt gga ttg aaa cgg ctg cct gat ttg att aag gaa gta ctg agc      1584
   Met Leu Gly Leu Lys Arg Leu Pro Asp Leu Ile Lys Glu Val Leu Ser
           515                 520                 525
   atg gat gac gaa att cag aaa cta gca aca gaa ctt tat cat cag aag      1632
   Met Asp Asp Glu Ile Gln Lys Leu Ala Thr Glu Leu Tyr His Gln Lys
       530                 535                 540
   tca gtt ctg ata atg gga cga ggc tat cat tat gct act tgt ctt gaa      1680
   Ser Val Leu Ile Met Gly Arg Gly Tyr His Tyr Ala Thr Cys Leu Glu
   545                 550                 555                 560
   ggg gca ctg aaa atc aaa gaa att act tat atg cac tct gaa ggc atc      1728
   Gly Ala Leu Lys Ile Lys Glu Ile Thr Tyr Met His Ser Glu Gly Ile
                   565                 570                 575
   ctt gct ggt gaa ttg aaa cat ggc cct ctg gct ttg gtg gat aaa ttg      1776
   Leu Ala Gly Glu Leu Lys His Gly Pro Leu Ala Leu Val Asp Lys Leu
               580                 585                 590
   atg cct gtg atc atg atc atc atg aga gat cac act tat gcc aag tgt      1824
   Met Pro Val Ile Met Ile Ile Met Arg Asp His Thr Tyr Ala Lys Cys
           595                 600                 605
   cag aat gct ctt cag caa gtg gtt gct cgg cag ggg cgg cct gtg gta      1872
   Gln Asn Ala Leu Gln Gln Val Val Ala Arg Gln Gly Arg Pro Val Val
       610                 615                 620
   att tgt gat aag gag gat act gag acc att aag aac aca aaa aga acg      1920
   Ile Cys Asp Lys Glu Asp Thr Glu Thr Ile Lys Asn Thr Lys Arg Thr
   625                 630                 635                 640
   atc aag gtg ccc cac tca gtg gac tgc ttg cag ggc att ctc agc gtg      1968
   Ile Lys Val Pro His Ser Val Asp Cys Leu Gln Gly Ile Leu Ser Val
                   645                 650                 655
   atc cct tta cag ttg ctg gct ttc cac ctt gct gtg ctg aga ggc tat      2016
   Ile Pro Leu Gln Leu Leu Ala Phe His Leu Ala Val Leu Arg Gly Tyr
               660                 665                 670
   gat gtt gat ttc cca cgg aat ctt gcc aaa tct gtg act gta gag tga      2064
   Asp Val Asp Phe Pro Arg Asn Leu Ala Lys Ser Val Thr Val Glu
           675                 680                 685
<210> 8
   <211> 687
   <212 > PRT
   <213> Séquence artificielle
<220>
   <221> misc_feature
   <222> (57)..(57)
   <223> Le 'Xaa' en position 57 représente Thr ou Ile.
<220>
   <223> GFAT1 modifiée par une étiquette de purification interne
<400> 8
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Xaa Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
                   85                  90                  95
   Ala Thr His Gly Glu Pro Ser Pro Val Asn Ser His Pro Gln Arg Ser
   100 105 110
   Asp Lys Asn Asn Glu Phe Ile Val Ile His Asn Gly Ile Ile Thr Asn
   115 120 125
   Tyr Lys Asp Leu Lys Lys Phe Leu Glu Ser Lys Gly Tyr Asp Phe Glu
   130 135 140
   Ser Glu Thr Asp Thr Glu Thr Ile Ala Lys Leu Val Lys Tyr Met Tyr
   145 150 155 160
   Asp Asn Arg Glu Ser Gln Asp Thr Ser Phe Thr Thr Leu Val Glu Arg
   165 170 175
   Val Ile Gln Gln Leu Glu Gly Ala Phe Ala Leu Val Phe Lys Ser Val
   180 185 190
   His Phe Pro Gly Gln Ala Val Gly Thr Arg Arg Gly Ser Pro Leu Leu
   195 200 205
   Ile Gly Val Arg Ser Glu His Lys Leu Ser Thr Asp His Ile Pro Ile
   210 215 220
   Leu Tyr Arg Thr Gly Lys Asp Lys Lys Gly Ser Cys Asn Leu Ser Arg
   225 230 235 240
   Val Asp Ser Thr Thr Cys Leu Phe Pro Val Glu Glu Lys Ala Val Glu
   245 250 255
   Tyr Tyr Phe Ala Ser Asp Ala Ser Ala Val Ile Glu His Thr Asn Arg
   260 265 270
   Val Ile Phe Leu Glu Asp Asp Asp Val Ala Ala Val Val Asp Gly Arg
   275 280 285
   Leu Ser Ile His Arg Ile Lys Arg Thr Ala Gly His His His His His
   290 295 300
   His Asp His Pro Gly Arg Ala Val Gln Thr Leu Gln Met Glu Leu Gln
   305 310 315 320
   Gln Ile Met Lys Gly Asn Phe Ser Ser Phe Met Gln Lys Glu Ile Phe
   325 330 335
   Glu Gln Pro Glu Ser Val Val Asn Thr Met Arg Gly Arg Val Asn Phe
   340 345 350
   Asp Asp Tyr Thr Val Asn Leu Gly Gly Leu Lys Asp His Ile Lys Glu
   355 360 365
   Ile Gln Arg Cys Arg Arg Leu Ile Leu Ile Ala Cys Gly Thr Ser Tyr
   370 375 380
   His Ala Gly Val Ala Thr Arg Gln Val Leu Glu Glu Leu Thr Glu Leu
   385 390 395 400
   Pro Val Met Val Glu Leu Ala Ser Asp Phe Leu Asp Arg Asn Thr Pro
   405 410 415
   Val Phe Arg Asp Asp Val Cys Phe Phe Leu Ser Gln Ser Gly Glu Thr
   420 425 430
   Ala Asp Thr Leu Met Gly Leu Arg Tyr Cys Lys Glu Arg Gly Ala Leu
   435 440 445
   Thr Val Gly Ile Thr Asn Thr Val Gly Ser Ser Ile Ser Arg Glu Thr
   450 455 460
   Asp Cys Gly Val His Ile Asn Ala Gly Pro Glu Ile Gly Val Ala Ser
   465 470 475 480
   Thr Lys Ala Tyr Thr Ser Gln Phe Val Ser Leu Val Met Phe Ala Leu
   485 490 495
   Met Met Cys Asp Asp Arg Ile Ser Met Gln Glu Arg Arg Lys Glu Ile
   500 505 510
   Met Leu Gly Leu Lys Arg Leu Pro Asp Leu Ile Lys Glu Val Leu Ser
   515 520 525
   Met Asp Asp Glu Ile Gln Lys Leu Ala Thr Glu Leu Tyr His Gln Lys
   530 535 540
   Ser Val Leu Ile Met Gly Arg Gly Tyr His Tyr Ala Thr Cys Leu Glu
   545 550 555 560
   Gly Ala Leu Lys Ile Lys Glu Ile Thr Tyr Met His Ser Glu Gly Ile
   565 570 575
   Leu Ala Gly Glu Leu Lys His Gly Pro Leu Ala Leu Val Asp Lys Leu
   580 585 590
   Met Pro Val Ile Met Ile Ile Met Arg Asp His Thr Tyr Ala Lys Cys
   595 600 605
   Gln Asn Ala Leu Gln Gln Val Val Ala Arg Gln Gly Arg Pro Val Val
   610 615 620
   Ile Cys Asp Lys Glu Asp Thr Glu Thr Ile Lys Asn Thr Lys Arg Thr
   625 630 635 640
   Ile Lys Val Pro His Ser Val Asp Cys Leu Gln Gly Ile Leu Ser Val
   645 650 655
   Ile Pro Leu Gln Leu Leu Ala Phe His Leu Ala Val Leu Arg Gly Tyr
   660 665 670
   Asp Val Asp Phe Pro Arg Asn Leu Ala Lys Ser Val Thr Val Glu
   675 680 685
<210> 9
   <211> 2067
   <212> ADN
   <213> Séquence artificielle
<220>
   <223> GFAT2 modifiée par une étiquette de purification interne
<220>
   <221> CDS
   <222> (1)..(2067)
   <223>
<400> 9
   atg tgc gga atc ttt gcc tac atg aac tac aga gtc ccc cgg acg agg      48
   aag gag atc ttc gaa acc ctc atc aag ggc ctg cag cgg ctg gag tac      96
   aga ggc tac gac tcg gca ggt gtg gcg atc gat ggg aat aat cac gaa     144
   gtc aaa gaa aga cac att cag ctg gtc aag aaa agg ggg aaa gtc aag     192
   gct ctc gat gaa gaa ctt tac aaa caa gac agc atg gac tta aaa gtg     240
   gag ttt gag aca cac ttc ggc att gcc cac acg cgc tgg gcc acc cac     288
   ggg gtc ccc agt gct gtc aac agc cac cct cag cgc tca gac aaa ggc     336
   aac gaa ttt gtt gtc atc cac aat ggg atc atc aca aat tac aaa gat     384
   ctg agg aaa ttt ctg gaa agc aaa ggc tac gag ttt gag tca gaa aca     432
   gat aca gag acc atc gcc aag ctg att aaa tat gtg ttc gac aac aga      480
   gaa act gag gac att acg ttt tca acg ttg gtc gag aga gtc att cag      528
   cag ttg gaa ggt gca ttc gcg ctg gtt ttc aag agt gtc cac tac cca      576
   gga gaa gcc gtt gcc aca cgg aga ggc agc ccc ctg ctc atc gga gtc      624
   cgg agc aaa tac aag ctc tcc aca gaa cag atc cct atc tta tac agg      672
   acg tgc act ctg gag aat gtg aag aat atc tgt aag aca cgg atg aag      720
   agg ctg gac agc tcc gcc tgc ctg cat gct gtg ggc gac aag gcc gtg      768
   gaa ttc ttc ttt gct tct gat gca agc gct atc ata gag cac acc aac      816
   cgg gtc atc ttc ctg gag gac gat gac atc gcc gca gtg gct gat ggg      864
   aaa ctc tcc att cac cgg gtc aag cgc tcg gcc agt cat cac cat cac      912
   cat cac gat gac cca tct cga gcc atc cag acc ttg cag atg gaa ctg      960
   cag caa atc atg aaa ggt aac ttc agt gcg ttt atg cag aag gag atc     1008
   ttc gaa cag cca gaa tca gtt ttc aat act atg aga ggt cgg gtg aat     1056
   ttt gaa acc aac aca gtg ctc ctg ggt ggc ttg aag gac cac ttg aag     1104
   gag att cga cga tgc cga cgg ctc atc gtg att ggc tgt gga acc agc     1152
   tac cac gct gcc gtg gct acg cgg caa gtt ttg gag gaa ctg act gag     1200
   ctt cct gtg atg gtt gaa ctt gct agt gat ttt ctg gac agg aac aca       1248
   cct gtg ttc agg gat gac gtt tgc ttt ttc atc agc cag tca ggc gag       1296
   acc gcg gac acc ctc ctg gcg ctg cgc tac tgt aag gac cgc ggc gct       1344
   ctc acc gtg ggc gtc acc aac acc gtg ggc agc tcc atc tct cgc gag       1392
   acc gac tgc ggc gtc cac atc aac gca ggg ccg gag gtc ggc gtg gcc       1440
   agc acc aag gct tat acc agt cag ttc atc tct ctg gtg atg ttt ggt       1488
   ttg atg atg tct gaa gac cga att tca cta caa aac agg agg caa gag       1536
   atc atc cgt ggc ttg aga tct tta cct gag ctg atc aag gaa gtg ctg       1584
   tct ctg gag gag aag atc cac gac ttg gcc ctg gag ctc tac acg cag       1632
   aga tcg ctg ctg gtg atg ggg cgg ggc tac aac tat gcc acc tgc ctg       1680
   gaa gga gcc ctg aaa att aaa gag ata acc tac atg cac tca gaa ggc       1728
   atc ctg gct ggg gag ctg aag cac ggg ccc ctg gca ctg att gac aag       1776
   cag atg ccc gtc atc atg gtc att atg aag gat cct tgc ttc gcc aaa       1824
   tgc cag aac gcc ctg cag caa gtc acg gcc cgc cag ggt cgc ccc att       1872
   ata ctg tgc tcc aag gac gat act gaa agt tcc aag ttt gcg tat aag       1920
   aca atc gag ctg ccc cac act gtg gac tgc ctc cag ggc atc ctg agc       1968
   gtg att ccg ctg cag ctg ctg tcc ttc cac ctg gct gtt ctc cga gga      2016
   tat gac gtt gac ttc ccc aga aat ctg gcc aag tct gta act gtg gaa      2064
   tga                                                                  2067
<210> 10
   <211> 688
   <212> PRT
   <213> Séquence artificielle
<220>
   <223> GFAT2 modifiée par une étiquette de purification interne
<400> 10
<210> 11
   <211> 2118
   <212> ADN
   <213> Séquence artificielle
<220>
   <223> GPAT1Alt modifiée par une étiquette de purification interne
<220>
   <221> CDS
   <222> (1) .. (2118)
   <223>
<220>
   <221> misc_feature
   <222> (170)..(170)
   <223> t ou c
<400> 11
   atg tgt ggt ata ttt gct tac tta aac tac cat gtt cct cga acg aga      48
   Met Cys Gly Ile Phe Ala Tyr Leu Asn Tyr His Val Pro Arg Thr Arg
   1               5                   10                  15
   cga gaa atc ctg gag acc cta atc aaa ggc ctt cag aga ctg gag tac      96
   Arg Glu Ile Leu Glu Thr Leu Ile Lys Gly Leu Gln Arg Leu Glu Tyr
               20                  25                  30
   aga gga tat gat tct gct ggt gtg gga ttt gat gga ggc aat gat aaa     144
   Arg Gly Tyr Asp Ser Ala Gly Val Gly Phe Asp Gly Gly Asn Asp Lys
           35                  40                  45
   gat tgg gaa gcc aat gcc tgc aaa anc cag ctt att aag aag aaa gga     192
   Asp Trp Glu Ala Asn Ala Cys Lys Xaa Gln Leu Ile Lys Lys Lys Gly
       50                  55                  60
   aaa gtt aag gca ctg gat gaa gaa gtt cac aag caa caa gat atg gat     240
   Lys Val Lys Ala Leu Asp Glu Glu Val His Lys Gln Gln Asp Met Asp
   65                  70                  75                  80
   ttg gat ata gaa ttt gat gta cac ctt gga ata gct cat acc cgt tgg     288
   Leu Asp Ile Glu Phe Asp Val His Leu Gly Ile Ala His Thr Arg Trp
                   85                  90                  95
   gca aca cat gga gaa ccc agt cct gtc aat agc cac ccc cag cgc tct     336
   Ala Thr His Gly Glu Pro Ser Pro Val Asn Ser His Pro Gln Arg Ser
               100                 105                 110
   gat aaa aat aat gaa ttt atc gtt att cac aat gga atc atc acc aac     384
   Asp Lys Asn Asn Glu Phe Ile Val Ile His Asn Gly Ile Ile Thr Asn
           115                 120                 125
   tac aaa gac ttg aaa aag ttt ttg gaa agc aaa ggc tat gac ttc gaa     432
   Tyr Lys Asp Leu Lys Lys Phe Leu Glu Ser Lys Gly Tyr Asp Phe Glu
       130                 135                 140
   tct gaa aca gac aca gag aca att gcc aag ctc gtt aag tat atg tat     480
   Ser Glu Thr Asp Thr Glu Thr Ile Ala Lys Leu Val Lys Tyr Met Tyr
   145                 150                 155                 160
   gac aat cgg gaa agt caa gat acc agc ttt act acc ttg gtg gag aga     528
   Asp Asn Arg Glu Ser Gln Asp Thr Ser Phe Thr Thr Leu Val Glu Arg
                   165                 170                 175
   gtt atc caa caa ttg gaa ggt gct ttt gca ctt gtg ttt aaa agt gtt     576
   Val Ile Gln Gln Leu Glu Gly Ala Phe Ala Leu Val Phe Lys Ser Val
                   180                     185                     190
   cat ttt ccc ggg caa gca gtt ggc aca agg cga ggt agc cct ctg ttg        624
   His Phe Pro Gly Gln Ala Val Gly Thr Arg Arg Gly Ser Pro Leu Leu
           195                     200                     205
   att ggt gta cgg agt gaa cat aaa ctt tct act gat cac att cct ata        672
   Ile Gly Val Arg Ser Glu His Lys Leu Ser Thr Asp His Ile Pro Ile
       210                     215                     220
   ctc tac aga aca gct agg act cag att gga tca aaa ttc aca cgg tgg        720
   Leu Tyr Arg Thr Ala Arg Thr Gln Ile Gly Ser Lys Phe Thr Arg Trp
   225                     230                     235                     240
   gga tca cag gga gaa aga ggc aaa gac aag aaa gga agc tgc aat ctc        768
   Gly Ser Gln Gly Glu Arg Gly Lys Asp Lys Lys Gly Ser Cys Asn Leu
                   245                     250                     255
   tct cgt gtg gac agc aca acc tgc ctt ttc ccg gtg gaa gaa aaa gca        816
   Ser Arg Val Asp Ser Thr Thr Cys Leu Phe Pro Val Glu Glu Lys Ala
               260                     265                     270
   gtg gag tat tac ttt gct tct gat gca agt gct gtc ata gaa cac acc        864
   Val Glu Tyr Tyr Phe Ala Ser Asp Ala Ser Ala Val Ile Glu His Thr
           275                     280                     285
   aat cgc gtc atc ttt ctg gaa gat gat gat gtt gca gca gta gtg gat        912
   Asn Arg Val Ile Phe Leu Glu Asp Asp Asp Val Ala Ala Val Val Asp
       290                     295                     300
   gga cgt ctt tct atc cat cga att aaa cga act gca gga cat cac cat        960
   Gly Arg Leu Ser Ile His Arg Ile Lys Arg Thr Ala Gly His His His
   305                     310                     315                     320
   cac cat cac gat cac ccc gga cga gct gtg caa aca ctc cag atg gaa       1008
   His His His Asp His Pro Gly Arg Ala Val Gln Thr Leu Gln Met Glu
                   325                     330                     335
   ctc cag cag atc atg aag ggc aac ttc agt tca ttt atg cag aag gaa       1056
   Leu Gln Gln Ile Met Lys Gly Asn Phe Ser Ser Phe Met Gln Lys Glu
               340                     345                     350
   ata ttt gag cag cca gag tct gtc gtg aac aca atg aga gga aga gtc       1104
   Ile Phe Glu Gln Pro Glu Ser Val Val Asn Thr Met Arg Gly Arg Val
           355                     360                     365
   aac ttt gat gac tat act gtg aat ttg ggt ggt ttg aag gat cac ata       1152
   Asn Phe Asp Asp Tyr Thr Val Asn Leu Gly Gly Leu Lys Asp His Ile
       370                     375                     380
   aag gag atc cag aga tgc cgg cgt ttg att ctt att gct tgt gga aca       1200
   Lys Glu Ile Gln Arg Cys Arg Arg Leu Ile Leu Ile Ala Cys Gly Thr
   385                     390                     395                     400
   agt tac cat gct ggt gta gca aca cgt caa gtt ctt gag gag ctg act       1248
   Ser Tyr His Ala Gly Val Ala Thr Arg Gln Val Leu Glu Glu Leu Thr
                   405                     410                     415
   gag ttg cct gtg atg gtg gaa cta gca agt gac ttc ctg gac aga aac       1296
   Glu Leu Pro Val Met Val Glu Leu Ala Ser Asp Phe Leu Asp Arg Asn
               420                     425                     430
   aca cca gtc ttt cga gat gat gtt tgc ttt ttc ctt agt caa tca ggt       1344
   Thr Pro Val Phe Arg Asp Asp Val Cys Phe Phe Leu Ser Gln Ser Gly
           435                     440                     445
   gag aca gca gat act ttg atg ggt ctt cgt tac tgt aag gag aga gga      1392
   Glu Thr Ala Asp Thr Leu Met Gly Leu Arg Tyr Cys Lys Glu Arg Gly
       450                 455                 460
   gct tta act gtg ggg atc aca aac aca gtt ggc agt tcc ata tca cgg      1440
   Ala Leu Thr Val Gly Ile Thr Asn Thr Val Gly Ser Ser Ile Ser Arg
   465                 470                 475                 480
   gag aca gat tgt gga gtt cat att aat gct ggt cct gag att ggt gtg      1488
   Glu Thr Asp Cys Gly Val His Ile Asn Ala Gly Pro Glu Ile Gly Val
                   485                 490                 495
   gcc agt aca aag gct tat acc agc cag ttt gta tcc ctt gtg atg ttt      1536
   Ala Ser Thr Lys Ala Tyr Thr Ser Gln Phe Val Ser Leu Val Met Phe
               500                 505                 510
   gcc ctt atg atg tgt gat gat cgg atc tcc atg caa gaa aga cgc aaa      1584
   Ala Leu Met Met Cys Asp Asp Arg Ile Ser Met Gln Glu Arg Arg Lys
           515                 520                 525
   gag atc atg ctt gga ttg aaa cgg ctg cct gat ttg att aag gaa gta      1632
   Glu Ile Met Leu Gly Leu Lys Arg Leu Pro Asp Leu Ile Lys Glu Val
       530                 535                 540
   ctg agc atg gat gac gaa att cag aaa cta gca aca gaa ctt tat cat      1680
   Leu Ser Met Asp Asp Glu Ile Gln Lys Leu Ala Thr Glu Leu Tyr His
   545                 550                 555                 560
   cag aag tca gtt ctg ata atg gga cga ggc tat cat tat gct act tgt      1728
   Gln Lys Ser Val Leu Ile Met Gly Arg Gly Tyr His Tyr Ala Thr Cys
                   565                 570                 575
   ctt gaa ggg gca ctg aaa atc aaa gaa att act tat atg cac tct gaa      1776
   Leu Glu Gly Ala Leu Lys Ile Lys Glu Ile Thr Tyr Met His Ser Glu
               580                 585                 590
   ggc atc ctt gct ggt gaa ttg aaa cat ggc cct ctg gct ttg gtg gat      1824
   Gly Ile Leu Ala Gly Glu Leu Lys His Gly Pro Leu Ala Leu Val Asp
           595                 600                 605
   aaa ttg atg cct gtg atc atg atc atc atg aga gat cac act tat gcc      1872
   Lys Leu Met Pro Val Ile Met Ile Ile Met Arg Asp His Thr Tyr Ala
       610                 615                 620
   aag tgt cag aat gct ctt cag caa gtg gtt gct cgg cag ggg cgg cct      1920
   Lys Cys Gln Asn Ala Leu Gln Gln Val Val Ala Arg Gln Gly Arg Pro
   625                 630                 635                 640
   gtg gta att tgt gat aag gag gat act gag acc att aag aac aca aaa      1968
   Val Val Ile Cys Asp Lys Glu Asp Thr Glu Thr Ile Lys Asn Thr Lys
                   645                 650                 655
   aga acg atc aag gtg ccc cac tca gtg gac tgc ttg cag ggc att ctc      2016
   Arg Thr Ile Lys Val Pro His Ser Val Asp Cys Leu Gln Gly Ile Leu
               660                 665                 670
   agc gtg atc cct tta cag ttg ctg gct ttc cac ctt gct gtg ctg aga      2064
   Ser Val Ile Pro Leu Gln Leu Leu Ala Phe His Leu Ala Val Leu Arg
           675                 680                 685
   ggc tat gat gtt gat ttc cca cgg aat ctt gcc aaa tct gtg act gta      2112
   Gly Tyr Asp Val Asp Phe Pro Arg Asn Leu Ala Lys Ser Val Thr Val
       690                 695                 700
   gag tga                                                                 2118
   Glu
   705
<210> 12
   <211> 705
   <212> PRT
   <213> Séquence artificielle
<220>
   <221> misc_feature
   <222> (57)..(57)
   <223> Le 'Xaa' en position 57 représente Thr ou Ile.
<220>
   <223> GFAT1Alt modifiée par une étiquette de purification interne
<400> 12
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Xaa Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
                   85                  90                  95
   Ala Thr His Gly Glu Pro Ser Pro Val Asn Ser His Pro Gln Arg Ser
               100                 105                 110
   Asp Lys Asn Asn Glu Phe Ile Val Ile His Asn Gly Ile Ile Thr Asn
           115                 120                 125
   Tyr Lys Asp Leu Lys Lys Phe Leu Glu Ser Lys Gly Tyr Asp Phe Glu
       130                 135                 140
   Ser Glu Thr Asp Thr Glu Thr Ile Ala Lys Leu Val Lys Tyr Met Tyr
   145                 150                 155                 160
   Asp Asn Arg Glu Ser Gln Asp Thr Ser Phe Thr Thr Leu Val Glu Arg
                   165                 170                 175
   Val Ile Gln Gln Leu Glu Gly Ala Phe Ala Leu Val Phe Lys Ser Val
   180 185 190
   His Phe Pro Gly Gln Ala Val Gly Thr Arg Arg Gly Ser Pro Leu Leu
   195 200 205
   Ile Gly Val Arg Ser Glu His Lys Leu Ser Thr Asp His Ile Pro Ile
   210 215 220
   Leu Tyr Arg Thr Ala Arg Thr Gln Ile Gly Ser Lys Phe Thr Arg Trp
   225 230 235 240
   Gly Ser Gln Gly Glu Arg Gly Lys Asp Lys Lys Gly Ser Cys Asn Leu
   245 250 255
   Ser Arg Val Asp Ser Thr Thr Cys Leu Phe Pro Val Glu Glu Lys Ala
   260 265 270
   Val Glu Tyr Tyr Phe Ala Ser Asp Ala Ser Ala Val Ile Glu His Thr
   275 280 285
   Asn Arg Val Ile Phe Leu Glu Asp Asp Asp Val Ala Ala Val Val Asp
   290 295 300
   Gly Arg Leu Ser Ile His Arg Ile Lys Arg Thr Ala Gly His His His
   305 310 315 320
   His His His Asp His Pro Gly Arg Ala Val Gln Thr Leu Gln Met Glu
   325 330 335
   Leu Gln Gln Ile Met Lys Gly Asn Phe Ser Ser Phe Met Gln Lys Glu
   340 345 350
   Ile Phe Glu Gln Pro Glu Ser Val Val Asn Thr Met Arg Gly Arg Val
   355 360 365
   Asn Phe Asp Asp Tyr Thr Val Asn Leu Gly Gly Leu Lys Asp His Ile
   370 375 380
   Lys Glu Ile Gln Arg Cys Arg Arg Leu Ile Leu Ile Ala Cys Gly Thr
   385 390 395 400
   Ser Tyr His Ala Gly Val Ala Thr Arg Gln Val Leu Glu Glu Leu Thr
   405 410 415
   Glu Leu Pro Val Met Val Glu Leu Ala Ser Asp Phe Leu Asp Arg Asn
   420 425 430
   Thr Pro Val Phe Arg Asp Asp Val Cys Phe Phe Leu Ser Gln Ser Gly
   435 440 445
   Glu Thr Ala Asp Thr Leu Met Gly Leu Arg Tyr Cys Lys Glu Arg Gly
   450 455 460
   Ala Leu Thr Val Gly Ile Thr Asn Thr Val Gly Ser Ser Ile Ser Arg
   465 470 475 480
   Glu Thr Asp Cys Gly Val His Ile Asn Ala Gly Pro Glu Ile Gly Val
   485 490 495
   Ala Ser Thr Lys Ala Tyr Thr Ser Gln Phe Val Ser Leu Val Met Phe
   500 505 510
   Ala Leu Met Met Cys Asp Asp Arg Ile Ser Met Gln Glu Arg Arg Lys
   515 520 525
   Glu Ile Met Leu Gly Leu Lys Arg Leu Pro Asp Leu Ile Lys Glu Val
   530 535 540
   Leu Ser Met Asp Asp Glu Ile Gln Lys Leu Ala Thr Glu Leu Tyr His
   545 550 555 560
   Gln Lys Ser Val Leu Ile Met Gly Arg Gly Tyr His Tyr Ala Thr Cys
   565 570 575
   Leu Glu Gly Ala Leu Lys Ile Lys Glu Ile Thr Tyr Met His Ser Glu
   580 585 590
   Gly Ile Leu Ala Gly Glu Leu Lys His Gly Pro Leu Ala Leu Val Asp
   595 600 605
   Lys Leu Met Pro Val Ile Met Ile Ile Met Arg Asp His Thr Tyr Ala
   610 615 620
   Lys Cys Gln Asn Ala Leu Gln Gln Val Val Ala Arg Gln Gly Arg Pro
   625 630 635 640
   Val Val Ile Cys Asp Lys Glu Asp Thr Glu Thr Ile Lys Asn Thr Lys
   645 650 655
   Arg Thr Ile Lys Val Pro His Ser Val Asp Cys Leu Gln Gly Ile Leu
   660 665 670
   Ser Val Ile Pro Leu Gln Leu Leu Ala Phe His Leu Ala Val Leu Arg
   675 680 685
   Gly Tyr Asp Val Asp Phe Pro Arg Asn Leu Ala Lys Ser Val Thr Val
   690 695 700
   Glu
   705
<210> 13
   <211> 608
   <212> PRT
   <213> Escherichia coli
<400> 13
   Cys Gly Ile Val Gly Ala Ile Ala Gln Arg Asp Val Ala Glu Ile Leu
   1 5 10 15
   Leu Glu Gly Leu Arg Arg Leu Glu Tyr Arg Gly Tyr Asp Ser Ala Gly
   20 25 30
   Leu Ala Val Val Asp Ala Glu Gly His Met Thr Arg Leu Arg Arg Leu
   35 40 45
   Gly Lys Val Gln Met Leu Ala Gln Ala Ala Glu Glu His Pro Leu His
   50 55 60
   Gly Gly Thr Gly Ile Ala His Thr Arg Trp Ala Thr His Gly Glu Pro
   65 70 75 80
   Ser Glu Val Asn Ala His Pro His Val Ser Glu His Ile Val Val Val
   85 90 95
   His Asn Gly Ile Ile Glu Asn His Glu Pro Leu Arg Glu Glu Leu Lys
   100 105 110
   Ala Arg Gly Tyr Thr Phe Val Ser Glu Thr Asp Thr Glu Val Ile Ala
   115 120 125
   His Leu Val Asn Trp Glu Leu Lys Gln Gly Gly Thr Leu Arg Glu Ala
   130 135 140
   Val Leu Arg Ala Ile Pro Gln Leu Arg Gly Ala Tyr Gly Thr Val Ile
   145 150 155 160
   Met Asp Ser Arg His Pro Asp Thr Leu Leu Ala Ala Arg Ser Gly Ser
   165 170 175
   Pro Leu Val Ile Gly Leu Gly Met Gly Glu Asn Phe Ile Ala Ser Asp
   180 185 190
   Gln Leu Ala Leu Leu Pro Val Thr Arg Arg Phe Ile Phe Leu Glu Glu
   195 200 205
   Gly Asp Ile Ala Glu Ile Thr Arg Arg Ser Val Asn Ile Phe Asp Lys
           210                     215                     220
   Thr Gly Ala Glu Val Lys Arg Gln Asp Ile Glu Ser Asn Leu Gln Tyr
   225                 230                 235                 240
   Asp Ala Gly Asp Lys Gly Ile Tyr Arg His Tyr Met Gln Lys Glu Ile
                   245                 250                 255
   Tyr Glu Gln Pro Asn Ala Ile Lys Asn Thr Leu Thr Gly Arg Ile Ser
               260                 265                 270
   His Gly Gln Val Asp Leu Ser Glu Leu Gly Pro Asn Ala Asp Glu Leu
           275                 280                 285
   Leu Ser Lys Val Glu His Ile Gln Ile Leu Ala Cys Gly Thr Ser Tyr
       290                 295                 300
   Asn Ser Gly Met Val Ser Arg Tyr Trp Phe Glu Ser Leu Ala Gly Ile
   305                 310                 315                 320
   Pro Cys Asp Val Glu Ile Ala Ser Glu Phe Arg Tyr Arg Lys Ser Ala
                   325                 330                 335
   Val Arg Arg Asn Ser Leu Met Ile Thr Leu Ser Gln Ser Gly Glu Thr
               340                 345                 350
   Ala Asp Thr Leu Ala Gly Leu Arg Leu Ser Lys Glu Leu Gly Tyr Leu
           355                 360                 365
   Gly Ser Leu Ala Ile Cys Asn Val Pro Gly Ser Ser Leu Val Arg Glu
       370                 375                 380
   Ser Asp Leu Ala Leu Met Thr Asn Ala Gly Thr Glu Ile Gly Val Ala
   385                 390                 395                 400
   Ser Thr Lys Ala Phe Thr Thr Gln Leu Thr Val Leu Leu Met Leu Val
                   405                 410                 415
   Ala Lys Leu Ser Arg Leu Lys Gly Leu Asp Ala Ser Ile Glu His Asp
               420                 425                 430
   Ile Val His Gly Leu Gln Ala Leu Pro Ser Arg Ile Glu Gln Met Leu
           435                 440                 445
   Ser Gln Asp Lys Arg Ile Glu Ala Leu Ala Glu Asp Phe Ser Asp Lys
       450                 455                 460
   His His Ala Leu Phe Leu Gly Arg Gly Asp Gln Tyr Pro Ile Ala Leu
   465                 470                 475                 480
   Glu Gly Ala Leu Lys Leu Lys Glu Ile Ser Tyr Ile His Ala Glu Ala
   485 490 495
   Tyr Ala Ala Gly Glu Leu Lys His Gly Pro Leu Ala Leu Ile Asp Ala
   500 505 510
   Asp Met Pro Val Ile Val Val Ala Pro Asn Asn Glu Leu Leu Glu Lys
   515 520 525
   Leu Lys Ser Asn Ile Glu Glu Val Arg Ala Arg Gly Gly Gln Leu Tyr
   530 535 540
   Val Phe Ala Asp Gln Asp Ala Gly Phe Val Ser Ser Asp Asn Met His
   545 550 555 560
   Ile Ile Glu Met Pro His Val Glu Glu Val Ile Ala Pro Ile Phe Tyr
   565 570 575
   Thr Val Pro Leu Gln Leu Leu Ala Tyr His Val Ala Leu Ile Lys Gly
   580 585 590
   Thr Asp Val Asp Gln Pro Arg Asn Leu Ala Lys Ser Val Thr Val Glu
   595 600 605
<210> 14
   <211> 72
   <212> ADN
   <213> Séquence artificielle
<220>
   <223> Amorce
<400> 14
   tggacgtctt tctatccatc gaattaaacg aactgcagga catcaccatc accatcacga 60
   tcaccccgga cg 72
<210> 15
   <211> 44
   <212> ADN
   <213> Séquence artificielle
<220>
   <223> Amorce
<400> 15
   caaagttgac tcttcctctc attgtgttca cgacagactc tggc 44
<210> 16
   <211> 43
   <212> ADN
   <213> Séquence artificielle
<220>
   <223> Amorce
<400> 16
   aatctagatt catgctcgag cggccgccag tgtgattgat atc 43
<210> 17
   <211> 36
   <212> ADN
   <213> Séquence artificielle
<220>
   <223> Amorce
<400> 17
   atttttatca gagcgctggg ggtggctatt gacagg 36
<210> 18
   <211> 8
   <212> PRT
   <213> Séquence artificielle
<220>
   <223> Etiquette FLAG
<400> 18
   Asp Thr Lys Asp Asp Asp Asp Lys
   1               5
<210> 19
   <211> 6
   <212> PRT
   <213> Séquence artificielle
<220>
   <223> Etiquette hexa-histidine
<400> 19
   His His His His His His
   1               5